# EUROPEAN PATENT APPLICATION

(11) **EP 4 617 373 A1**
(43) Date of publication of application: **17.09.2025**
(21) Application number: 23888651.9
(22) Date of filing: 06.11.2023
(51) Int. Cl.: C12Q 1/06, C12N 5/09, C12N 5/10, C12N 15/09, C12Q 1/37

(54) **EVALUATION METHOD OF ANTICANCER AGENT AND KIT FOR EVALUATING ANTICANCER AGENT**

(30) Priority: 08.11.2022 JP 2022178739
(71) Applicant: Toppan Holdings Inc., Tokyo 110-0016 (JP)
(72) Inventor: TSUKAMOTO Kei, Tokyo 110-0016 (JP); KITANO Shiro, Tokyo 110-0016 (JP)
(74) Representative: Plasseraud IP
(86) International application number: PCT/JP2023/039861
(87) International publication number: WO 2024/101303

(57) **Abstract**

An anticancer drug assessment method including the steps of: culturing a cell structure containing stromal cells in which a gene encoding a live cell marker protein has been knocked out and cancer cells in the presence of an anticancer drug; and assessing the anticancer drug using an expression level of the live cell marker protein as an index in this order, wherein in the assessment, the expression level of the live cell marker protein is measured using a culture supernatant, or in the assessment, a substance that fluoresces or produces color when reacted with the live cell marker protein is added, and the expression level of the live cell marker protein is measured based on the amount of fluorescence or coloration of the substance.

## Description

### [Technical Field]

The present invention relates to anticancer drug assessment methods and anticancer drug assessment kits.

The present application is based on and claims the benefit of priority from Japanese Patent Application No. 2022-178739 filed November 8, 2022, the content of which is incorporated herein by reference.

### [Background Art]

For selection of suitable anticancer drugs, in the development of anticancer drugs or in cancer therapy, the effects of anticancer drugs on cancer cells are assessed in an *in vitro* assay system. Further, the drug approval rate in Japanese pharmaceutical companies is as low as only 0.1%. In order to increase the success rate of drug approval, it is necessary to make an early decision on whether drug candidate substances are highly likely to have desired drug efficacy. Highly reliable drug efficacy assessment methods are required. In particular, restrictions on conventional animal models are considered to be one of the reasons that hinder the successful development of new drugs in pharmaceutical companies. Pharmaceutical companies have required drug evaluation models that reproduce environments closer to the *in vivo* environment, in place of animal models.

However, conventional anticancer drug assessment methods performed in *in vitro* systems sometimes give only a low score to drugs that are able to exhibit superior anticancer activity when administered into a living body. Thus, there is a problem in that the obtained assessment is not always linked to actual clinical effects. Therefore, conventional *in vitro* assessment methods sometimes fail to select anticancer drugs suitable for cancer therapy, and fail to improve therapeutic results against cancer.

To assess the drug efficacy of anticancer drugs, prediction of drug efficacy is performed by genetic testing in a clinical site. For example, KRAS gene mutations in colorectal cancer are predictive factors for the therapeutic effects of cetuximab. Moreover, EGFR gene mutations in lung cancer are predictive factors for the therapeutic effects of gefitinib. However, many molecular targeted drug agents are only useable for general gene mutations. Furthermore, there are many somatic mutations that have not yet been identified. Therefore, prediction of drug efficacy by genetic testing may not be sufficient to assess drug efficacy. Furthermore, in the research and development of anticancer drugs, even when the efficacy of a drug, among many drugs, is determined by a conventional assessment method, the obtained efficacy assessment does not match the actual clinical results in many cases. This is a serious obstacle to research and development.

A recent analysis of data from colorectal cancer patients has revealed the possibility that many of genes highly expressed in patients with poor outcome may be expressed in stromal cells. This possibility was investigated using data from mice transplanted with human cancer cells, and it was found that genes highly expressed in patients with poor outcome were not derived from the human cancer tissues, but from the mouse tissues surrounding the human cancer tissues (NPL 1). In particular, it is reported that abnormally activated special fibroblasts often appear in highly malignant cancer. They are called "cancer-associated fibroblasts (CAF)." CAF reportedly promotes angiogenesis, and growth and infiltration of cancer cells, etc. (NPL 2). Accordingly, in the cancer microenvironment (in other words, cancer cells and their surrounding environment *in vivo*), the influence of the stroma on cancer cells is very large and it is considered that an environment closer to the *in vivo* environment can be produced by allowing cancer cells to coexist with the stroma.

In recent years, in addition to the general cytotoxic anticancer drugs that have been used so far, molecular targeted drugs that can exhibit therapeutic effects more specifically on cancer cells and their surrounding tissues (collectively referred to as "solid tumors") have been attracting attention as cancer therapy drugs. Among such drugs, angiogenesis inhibitors such as Avastin (registered trademark) (also known as bevacizumab, manufactured by Genentech) approved in the U.S. in 2004, unlike conventional anticancer drugs that directly exhibit a cytocidal effect, indirectly exhibit anti-tumor effects by inhibiting angiogenesis around the solid tumors, thereby cutting the supply of nutrition to the cancer itself and reducing the cancer growth rate.

Angiogenesis inhibitors are drugs that suppress or inhibit angiogenesis by blocking protein signal pathways of factors essential for angiogenesis and their receptors, which are essential for angiogenesis. In therapeutic methods, angiogenesis inhibitors are often used not only alone, but also in combination with conventional cytotoxic anticancer drugs. Combination therapy with angiogenesis inhibitors and anticancer drugs is more effective than therapy using general cytotoxic anticancer drugs alone. In Japan, combination therapy with angiogenesis inhibitors and various drugs have been approved for patients with unresectable advanced or recurrent colorectal cancer.

In anticancer therapy, therapy in which drugs with different action mechanisms are administered in combination is commonly performed, in the expectation of achieving higher therapeutic effects than when the drugs are administered alone. When combined administration is performed, it is preferable to accurately assess how much higher the actual therapeutic effect of the combined administration will be than when the drugs are administered alone.

The inventors have developed a method for accurately assessing the drug efficacy of combination therapy with angiogenesis inhibitors and anticancer drugs using an *in vitro* system (PTL 1). In the above method, the drug efficacy of an anticancer drug is assessed using, as an indicator, the influence on cancer cells contained in a cell structure containing a stroma closer to the *in vivo* environment, or cancer cells separated from the cell structure containing the stroma by a semipermeable membrane. Therefore, highly reliable assessment can be obtained even in an *in vitro* assessment system.

### [Citation List]

### [Patent Literature]

PTL 1: WO 2017/183673

### [Non-Patent Literature]

NPL 1: Isella C et al., "Stromal contribution to the colorectal cancer transcriptome.", Nature Genetics, Vol. 47, Issue 4, pp. 312-319, 2015.
NPL 2: Shimoda M et al., "Carcinoma-associated fibroblasts are a rate-limiting determinant for tumour progression.", Seminars in Cell & Developmental Biology Vol. 21, Issue 1, pp. 19-25, 2010.

### [Summary of Invention]

### [Technical Problem]

However, in the method described in PTL 1 and the like, tissues are fixed after anticancer drug treatment, and only cancer cells are immunostained for assessment, which requires destruction of the tissues for assessment. There are also nondestructive assessment methods, such as introducing a gene encoding fluorescent protein into cancer cells in advance, but it is difficult to introduce a gene into all cells, and pre-treatment may not be successful due to cell differences.

The present invention has been made in view of the above circumstances and provides an accurate and nondestructive anticancer drug assessment method using an *in vitro* system, and an anticancer drug assessment kit for use in the method.

### [Solution to Problem]

That is, the present invention includes the following aspects.
(1) An anticancer drug assessment method including the steps of:
   culturing a cell structure containing stromal cells in which a gene encoding a live cell marker protein has been knocked out and cancer cells in the presence of an anticancer drug; and
   assessing the anticancer drug using an expression level of the live cell marker protein as an index in this order, wherein
   in the assessment, the expression level of the live cell marker protein is measured using a culture supernatant, or
   in the assessment, a substance that fluoresces or produces color when reacted with the live cell marker protein is added, and the expression level of the live cell marker protein is measured based on the amount of fluorescence or coloration of the substance.
(2) The anticancer drug assessment method according to (1), wherein the live cell marker protein is a serine protease.
(3) The anticancer drug assessment method according to (2), wherein the serine protease is tripeptidyl peptidase 1.
(4) The anticancer drug assessment method according to (2) or (3), wherein the substance is a substrate.
(5) An anticancer drug assessment kit including: a cell structure containing stromal cells in which a gene encoding a live cell marker protein has been knocked out; and a cell culture vessel that accommodates the cell structure.

### [Advantageous Effects of Invention]

According to the anticancer drug assessment method and anticancer drug assessment kit of the above aspects, accurate and nondestructive anticancer drug assessment can be performed using an *in vitro* system. Since it is a nondestructive assessment system, a larger amount of data can be obtained continuously from the same sample over a long period of time.

### [Description of Embodiments]

### <<Anticancer Drug Assessment Method>>

An anticancer drug assessment method of the present embodiment (hereinafter, also simply referred to as an "assessment method of the present embodiment") includes the steps of:
culturing a cell structure containing stromal cells in which a gene encoding a live cell marker protein has been knocked out and cancer cells in the presence of an anticancer drug (hereinafter, also simply referred to as a "culture step"); and
assessing the anticancer drug using an expression level of the live cell marker protein as an index (hereinafter, also simply referred to as an "anticancer drug assessment step") in this order.

In the anticancer drug assessment step, the expression level of the live cell marker protein is measured using a culture supernatant, or a substance that fluoresces or produces color when reacted with the live cell marker protein is added, and the expression level of the live cell marker protein is measured based on the amount of fluorescence or coloration of the substance.

Since the anticancer drug assessment is performed based on an expression level of a live cell marker protein, it can be said that an assessment method of the present embodiment is a method of measuring an expression level of a live cell marker protein, the method including the steps of:
culturing a cell structure containing stromal cells in which a gene encoding a live cell marker protein has been knocked out and cancer cells in the presence of an anticancer drug; and
measuring an expression level of the live cell marker protein in this order.

Conventional assessment methods are destructive, such as immunostaining that requires tissue fixation. On the other hand, the assessment method of the present embodiment enables nondestructive assessment of the efficacy of an anticancer drug without requiring tissue fixation. In addition, since the assessment method of the present embodiment is a nondestructive assessment system, data can be obtained continuously and over time while maintaining the culture of the cell structure. Therefore, according to the assessment method of the present embodiment, it is possible to assess components that behave quickly, such as metabolites of cancer cells.

Next, details of each step of the assessment method of the present embodiment will be described below.

### <Culture Step>

In the culture step, a cell structure containing stromal cells in which a gene encoding a live cell marker protein has been knocked out and cancer cells (hereinafter, also simply referred to as a "cell structure") in the presence of an anticancer drug.

Specifically, the cell structure is cultured in a culture medium containing one or more anticancer drugs.

The amount of anticancer drug contained in the culture medium can be experimentally determined in consideration of the culture conditions, such as the type of anticancer drug, the type and number of cells constituting the cell structure, the type and amount of cancer cells, the type of culture medium, culture temperature and culture time.

The culture time is not specifically limited, and may be, for example, 24 to 96 hours, preferably 48 to 96 hours, and more preferably 48 to 72 hours. If necessary, hydrodynamic loads, such as reflux, may be applied within a range that does not significantly change the culture environment.

### [Cell structure]

In the present invention and the present specification, the "cell structure" is a three-dimensional structure in which multiple cell layers are laminated. The cell structure used in the present embodiment contains stromal cells in which a gene encoding a live cell marker protein has been knocked out and cancer cells. The cell structure may be a cell structure in which cancer cells are scattered in the entire structure, or a cell structure in which cancer cells are present only in a specific layer.

Alternatively, the cell structure may be one in which a stromal cell layer and a cancer cell layer are divided by a semipermeable membrane so that components secreted from the stromal cells constituting the three-dimensional structure can be in contact with the cancer cells. That is, the cell structure may be one in which a layer containing cancer cells and a layer containing stromal cells are divided by a semipermeable membrane. In the cell structure, the layer containing cancer cells may be a single layer or a multilayer. Similarly, the layer containing stromal cells may be a single layer or a multilayer. Moreover, the cell layer containing stromal cells may be a layer formed only from one or more types of stromal cells, or a layer containing cells other than stromal cells. Similarly, the cell layer containing cancer cells may be a layer formed only from cancer cells, or a layer containing cells other than cancer cells.

Due to the use of a cell structure in which stromal cells are formed into a three-dimensional structure, such as *in vivo* stromal tissue, and components secreted from the stromal cells are able to contact cancer cells through a semipermeable membrane, the same environment as a cancer cell environment under the influence of *in vivo* stromal tissue can be reproduced, and *in vivo* anticancer activity can be appropriately assessed. Therefore, the use of the cell structure enables the assessment of anticancer drugs more accurately reflecting human clinical results even in an *in vitro* assessment system, and highly reliable assessment can be obtained.

When cancer cells are collected from a cancer patient, many fibroblasts are also collected along with the cancer cells, and it is difficult to separate the fibroblasts from the cancer cells. Examples of the method of removing fibroblasts from cancer tissue collected from a cancer patient include culturing all the cells of the cancer tissue in a serum-free medium to kill fibroblasts, and then separating the fibroblast masses from the cancer cells using a semipermeable membrane. However, there is a concern that once the cancer cells penetrate a semipermeable membrane, cell-cell contact is lost from the cancer cells, leading to a failure in normal drug assessment.

Accordingly, in many cases, a cell population containing cancer cells collected from a cancer patient is similarly labeled regardless of the cell type, and then used for production of cell structures. However, it is difficult to label cancer cells in isolation from fibroblasts, which may lead to an erroneous drug efficacy assessment when a cell population collected from a cancer patient contains many fibroblasts. In particular, since the cell structure used in the present embodiment contains many stromal cells, it is necessary to accurately assess the drug effects on the cancer cells targeted by the drug. In the assessment method according to the present embodiment, stromal cells constituting a layer containing stromal cells and stromal cells derived from a cancer patient are separated by a semipermeable membrane so that they are not mixed. Therefore, even if a cell structure contains many stromal cells together with cancer cells, the influence of the stromal cells derived from the cancer patient is suppressed, and more reliable assessment can be obtained.

### (Stromal Cells)

Stromal cells in which a gene encoding a live cell marker protein has been knocked out can be prepared by knocking out the gene encoding the live cell marker protein in the stromal cells using known gene modification techniques.

The live cell marker protein is not specifically limited as long as it is a protein expressed only in live cells, and expressed in both stromal cells and cancer cells, and the loss of the gene encoding the protein does not affect the survival of the stromal cells.

Specific examples of the live cell marker protein include A4GNT, AAAS, AADAC, ABCA1, ABCA2, ABCA3, ABCA7, ABCB1, ABCB11, ABCB4, ABCB7, ABCB8, ABCC1, ABCC2, ABCC3, ABCC4, ABCC5, ABCC6, ABCD2, ABCD3, ABCD4, ABCF2, ABCG1, ABCG2, ABRA, ACCN1, ACCN2, ACCN3, ACCN4, ACE, ACP2, ACP5, ACTL6A, ACTN1, ACTN2, ACTN3, ACVR1, ACVR1B, ACVR1C, ACVR2A, ACVR2B, ACVRL1, ADAM11, ADAM12, ADAM17, ADAM2, ADAM23, ADAM29, ADAM30, ADAM8, ADAM9, ADCY3, ADCY7, ADCY8, ADCY9, ADCYAP1R1, ADI1, ADORA1, ADORA2A, ADORA2B, ADORA3, ADRA1A, ADRA1B, ADRA1D, ADRA2A, ADRA2B, ADRA2C, ADRB1, ADRB2, ADRB3, ADRM1, AFTPH, AGER, AGPAT3, AGTR1, AGTR2, AGTRL1, AIFM2, AIFM3, AKAP7, AKT1, AKTIP, ALAS2, ALG3, ALK, ALOX12, AMBP, AMFR, AMOTL1, AMTN, ANKFY1, ANKH, ANKRA2, ANKRD20A1, ANPEP, ANXA2, AOC3, AP1G2, AP1GBP1, AP1S1, AP2S1, AP3S1, APC, APC2, APH1A, APH1B, APOM, APP, AQP1, AQP2, AQP4, AQP5, AQP6, AQP7, AQP8, AQP9, ARCN1, ARF1, ARF6, ARFGEF2, ARFIP1, ARFIP2, ARHGEF1, ARL6, ARL6IP5, ARRB1, ARRB2, ART1, ART3, ASGR1, ASH1L, ASPH, ATF6, ATP12A, ATP1A1, ATP1A2, ATP1A3, ATP1A4, ATP1B1, ATP1B3, ATP1B4, ATP2A2, ATP2B1, ATP2B3, ATP2B4, ATP2C1, ATP4A, ATP5A1, ATP5B, ATP5C1, ATP5D, ATP5E, ATP5G1, ATP5G2, ATP5G3, ATP5J, ATP5O, ATP6AP1, ATP6AP2, ATP6V0A4, ATP6V0B, ATP6V0C, ATP6V1A, ATP6V1B1, ATP6V1C1, ATP6V1E1, ATP6V1F, ATP7A, ATP7B, ATP8B1, ATRN, AVPR1A, AVPR1B, AVPR2, AXIN1, AXL, B3GAT1, B3GNT3, B4GALT1, B4GALT4, B4GALT7, BACE1, BAI1, BAI2, BAI3, BAIAP2, BAK1, BAMBI, BASP1, BAX, BCAM, BCAP31, BCAR1, BCL10, BCL2, BCL2L13, BCS1L, BDKRB1, BDKRB2, BEST1, BET1, BET1L, BFAR, BMPR1A, BMPR2, BNIP1, BNIP2, BNIP3, BNIP3L, BPI, BRS3, BSCL2, BSND, BST1, BST2, BTK, BTN1A1, BTN2A1, C11orf2, C1GALT1, C1QBP, C3AR1, C3orf31, C5AR1, C6orf25, C7, C8A, C8B, C9, C9orf127, C9orf58, CA12, CA14, CA9, CACNA1A, CACNA1B, CACNA1C, CACNA1D, CACNA1E, CACNA1F, CACNA1G, CACNA1H, CACNA1S, CACNA2D1, CACNB1, CACNB2, CACNB3, CACNB4, CACNG1, CACNG2, CACNG4, CADM1, CADM3, CALCRL, CALM3, CALY, CAPRIN1, CARD11, CASK, CASP7, CASR, CAV1, CAV2, CAV3, CBL, CBX5, CCBP2, CCDC70, CCDC8, CCDC88A, CCKAR, CCKBR, CCR1, CCR10, CCR2, CCR3, CCR4, CCR5, CCR6, CCR7, CCR8, CCR9, CCRL1, CCRL2, CD14, CD151, CD160, CD163, CD164, CD19, CD1B, CD1C, CD1D, CD200, CD22, CD226, CD24, CD247, CD28, CD300C, CD33, CD36, CD37, CD38, CD3E, CD4, CD40, CD40LG, CD46, CD47, CD48, CD5, CD52, CD53, CD55, CD6, CD63, CD68, CD69, CD7, CD70, CD72, CD74, CD79A, CD79B, CD81, CD82, CD83, CD84, CD8B, CD96, CD97, CD99, CDC42, CDC42BPA, CDC42BPB, CDC42EP2CDC42SE1, CDC42SE2, CDH1, CDH13, CDH15, CDH24, CDH5, CDK5, CDK5R1CEACAM4, CEACAM5, CEACAM6, CEACAM7, CEACAM8, CELSR1, CELSR3, CENPF, CENTA1, CENTA2, CEPT1, CFTR, CHL1, CHMP1A, CHRM1, CHRM2, CHRM3, CHRM4, CHRM5, CHRNA1, CHRNA2, CHRNA3, CHRNA4, CHRNA6, CHRNA7, CHRNB2, CHRNB3, CHRNB4, CHRND, CHRNE, CHRNG, CHST7, CIB1, CKAP4, CKLF, CLCA1, CLCA2, CLCA4, CLCN1, CLCN3, CLCN5, CLCNKA, CLCNKB, CLDN1, CLDN10, CLDN11, CLDN12, CLDN14, CLDN15, CLDN16, CLDN17, CLDN18, CLDN19, CLDN2, CLDN20, CLDN22, CLDN23, CLDN3, CLDN4, CLDN5, CLDN6, CLDN7, CLDN8, CLDN9, CLEC10A, CLEC1A, CLEC1B, CLEC2B, CLEC2D, CLEC4A, CLEC4M, CLEC5A, CLEC7A, CLN3, CLN5, CLNS1A, CLPTM1, CMKLR1, CNGA1, CNIH2, CNKSR1, CNN2, CNPY2, CNR1, CNR2, CNTFR, CNTN2, CNTNAP1, COG2, COL13A1, COL17A1, COLEC12, COPA, COPB1, COPB2, COPE, COPG, COPG2, COPZ1, CORIN, CORO1A, COX15, COX18, COX6B2, CPE, CPM, CR1, CRHR1, CRHR2, CRIM1, CRIPAK, CRNN, CRTAM, CSF1R, CSF2RA, CSF2RB, CSF3R, CSK, CSNK2A1, CSPG4, CSPG5, CTGF, CTLA4, CTNNA3, CTNNB1, CTNS, CUBN, CUTA, CUZD1, CX3CL1, CX3CR1, CXADR, CXCR3, CXCR4, CXCR5, CXCR6, CYB561, CYB5R2, CYB5R3, CYBB, CYSLTR1, DAB2, DAD1, DAG1, DARC, DC2, DCBLD2, DCLK1, DCT, DDOST, DDR1, DDR2, DDX19B, DEGS1, DERL1, DERL2, DERL3, DGKD, DGKK, DHCR7, DHRS9, DKK1, DLG1, DLG2, DLG4, DLK1, DMBT1, DMD, DNER, DNM2, DOLPP1, DOPEY1, DOPEY2, DPM1, DPM2, DPM3, DRD1, DRD2, DRD3, DRD4, DRD5, DSC1, DSC3, DSCAM, DST, DTNBP1, DULLARD, DYSF, EBI2, EBI3, EBP, ECE1, ECE2, ECEL1, EDA, EDEM1, EDG1, EDG2, EDG3, EDG4, EDG6, EDG7, EDNRA, EDNRB, EEA1, EFNA1, EFNA3, EFNA4, EFNA5, EFNB1, EFNB2, EFNB3, EGF, EGFR, EIF2B1, EIF5A, ELMO1, ELTD1, EMCN, EMD, EMP1, EMP2, EMR1, EMR2, EMR3, EMR4, ENG, ENOX2, ENPEP, ENPP1, ENPP2, ENPP3, ENTPD1, ENTPD4, EPB41, EPB41L2, EPB41L3, EPB42, EPC1, EPGN, EPHA1, EPHA2, EPHA3, EPHA4, EPHA5, EPHA8, EPHB1, EPHB2, EPHB3, EPHB4, EPOR, EPS15, ERBB2IP, ERBB3, ERBB4, ERC1, ERC2, EREG, ERLIN1, ERLIN2, ERMAP, ERN1, EVI2B, EVL, EXT1, EXT2, EZR, F11R, F2R, F2RL1, F2RL2, F2RL3, F3, FADS2, FAM84B, FASLG, FAT, FCAR, FCER1A, FCER1G, FCER2, FCGR3A, FDFT1, FER1L3, FFAR1, FFAR2, FFAR3, FGFR1, FGFR3, FGFR4, FIS1, FLOT1, FLT1, FLT3, FLT4, FLVCR1, FOLH1, FOLR1, FOLR3, FPR1, FPRL1, FPRL2, FRAG1, FRAP1, FRS2, FRZB, FSHR, FUT1, FUT7, FXYD1, FXYD3, FXYD5, FXYD6, FYN, FZD10, FZD2, FZD3, FZD4, FZD5, FZD6, FZD7, FZD8, FZD9, GABARAP, GABBR1, GABBR2, GABRA1, GABRA2, GABRA3, GABRA4, GABRA5, GABRA6, GABRB1, GABRB2, GABRB3, GABRD, GABRE, GABRG2, GABRQ, GABRR1, GABRR2, GAL3ST1, GAL3ST2, GALNT3, GALR1, GALR2, GALR3, GAS1, GBA2, GBAS, GCA, GCGR, GCNT1, GFRA2, GFRA3, GGCX, GGTLA1, GHR, GHRHR, GHSR, GIPC1, GIPR, GJA1, GJA4, GJA8, GJB1, GJB2, GJD3, GLE1, GLG1, GLP1R, GLP2R, GLRA1, GML, GNA11, GNA14, GNA15, GNAI1, GNAQ, GNAS, GNAZ, GNG4, GNRHR, GOLGA3, GOLGA5, GOLGB1, GOLIM4, GOLM1, GOSR1, GOT2, GP1BA, GP5, GP6, GP9, GPA33, GPAA1, GPC1, GPC3, GPC4, GPC5, GPC6, GPER, GPNMB, GPR1, GPR109B, GPR110, GPR111, GPR112, GPR113, GPR114, GPR115, GPR116, GPR12, GPR123, GPR124, GPR125, GPR126, GPR128, GPR133, GPR135, GPR137B, GPR143, GPR144, GPR15, GPR17, GPR175, GPR176, GPR182, GPR19, GPR20, GPR21, GPR22, GPR23, GPR25, GPR3, GPR31, GPR32, GPR34, GPR35, GPR37, GPR39, GPR4, GPR42, GPR44, GPR45, GPR50, GPR52, GPR55, GPR56, GPR6, GPR64, GPR65, GPR68, GPR75, GPR97, GPR98, GPRC5A, GPRC5C, GRB10, GRIA1, GRIA2, GRIA3, GRIA4, GRID2, GRIK1, GRIK2, GRIK3, GRIK4, GRIN1, GRIN2A, GRIN2B, GRIN2C, GRINL1A, GRM1, GRM2, GRM3, GRM4, GRM5, GRM6, GRM7, GRM8, GRPR, GUCY2D, GUCY2F, GYPA, GYPB, GYPC, GYPE, GZMA, GZMB, HAS1, HAS2, HAS3, HBEGF, HCCS, HCN2, HCRTR1, HCRTR2, HDAC11, HDLBP, HERPUD1, HFE, HLA-DOA, HLA-DRA, HLA-DRB3, HLA-DRB4, HLA-DRB5, HMGCR, HNRPM, HOMER1, HPN, HPS1, HRAS, HRB, HRH1, HRH2, HRH3, HS3ST1, HS3ST2, HS3ST3A1, HS3ST3B1, HS6ST1, HSD17B10, HSD17B2, HSD3B1, HSD3B2, HSP90B1, HTATIP2, HTR1A, HTR1B, HTR1D, HTR1E, HTR1F, HTR2A, HTR2B, HTR3A, HTR3B, HTR4, HTR5A, HTR6, HTR7, HTRA2, HVCN1, ICA1, ICAM1, ICAM2, ICAM3, ICAM4, ICAM5, IFITM1, IFITM3, IFNAR1, IFNAR2, IFNGR1, IFNGR2, IGF2R, IGSF1, IGSF2, IGSF6, IGSF8, IL10RA, IL10RB, IL11RA, IL12RB1, IL12RB2, IL13RA1, IL15, IL17RB, IL18R1, IL1R1, IL1RAP, IL1RL2, IL27RA, IL28RA, IL29, IL2RA, IL2RB, IL2RG, IL4R, IL5RA, IL6R, IL6ST, IL8RB, IL9R, IMMT, INSR, IPO7, IRS4, ITGA1, ITGA10, ITGA11, ITGA2, ITGA3, ITGA5, ITGA8, ITGA9, ITGAD, ITGAE, ITGAM, ITGAX, ITGB1, ITGB3, ITGB4, ITGB6, ITGB7, ITGB8, ITLN1, ITPR1, ITPR2, IPR3, IZUMO1, JAG2, JTB, KCNA1, KCNA2, KCNA3, KCNA4, KCNA5, KCNA6, KCNB2, KCNC1, KCNC3, KCNC4, KCND3, KCNE1, KCNE1L, KCNE2, KCNG2, KCNG3, KCNH1, KCNH2, KCNH4, KCNJ1, KCNJ10, KCNJ11, KCNJ14, KCNJ15, KCNJ2, KCNJ3, KCNJ4, KCNJ5, KCNJ6, KCNJ8, KCNK1, KCNK3, KCNK5, KCNK6, KCNMA1, KCNMB2, KCNMB3, KCNMB4, KCNN1, KCNN3, KCNN4, KCNQ1, KCNQ2, KCNQ3, KCNQ5, KCNS1, KDELR1, KDELR2, KDR, KEL, KHDRBS1, KIF1B, KIR2DL1, KIR2DL3, KIR2DL4, KIR2DS3, KIR2DS4, KIR3DL2, KIRREL3, KISSIR, KL, KLRA1, KLRB1, KLRC1, KLRC2, KLRD1, KLRF1, KLRG1, KLRK1, KPNA1, KPNA3, KPNB1, KRT1, KRTCAP2, KTN1, L2HGDH, L3MBTL4, LAMB1, LAMC3, LAMP1, LAMP2, LAMP3, LANCL1, LANCL2, LAPTM5, LARGE, LAT, LAT2, LAX1, LAYN, LBR, LCK, LCT, LDLR, LDLRAP1, LEMD3, LETM1, LGALS3, LGALS4, LGICZ1, LGR5, LHCGR, LIFR, LILRA3, LILRB1, LILRB2, LILRB3, LIMA1, LMAN1, LMAN2L, LMNB1, LMTK2, LNPEP, LPHN1, LPHN2, LPHN3, LRIT1, LRMP, LRP1, LRP12, LRP3, LRP5, LRP6, LRPAP1, LRRC32, LRRC4C, LSR, LST1, LTB4R, LTB4R2, LTC4S, LTK, LY6E, LY75, LY86, LY96, LYN, LYPD3, LYVE1, M6PR, MADD, MAEA, MAGEA1, MAGEE1, MAGI1, MAGT1, MAL, MALL, MAN1A2, MAN1B1, MAN1C1, MAP1LC3A, MAP1LC3B, MAP1LC3C, MAP3K12, MAP4K2, MAPT, MARCKSL1, MARCO, MAS1, MATR3, MBTPS2, MC1R, MC2R, MC3R, MC4R, MC5R, MCHR1, MCL1, MCTP1, MCTP2, MDGA1, MEP1A, MEP1B, MERTK, MET, MFI2, MFN2, MFRP, MGAT1, MGAT3, MIP, MIR16, MLANA, MLLT4, MLNR, MMD, MME, MMP14, MMP15, MMP16, MMP17, MMP24, MPL, MPP1, MPP2, MPP3, MPV17, MPZ, MPZL1, MRAS, MRC1, MRPL19, MRPL32, MRVI1, MS4A1, MS4A2, MSN, MSR1, MST1R, MSTO1, MTFR1, MTNR1A, MTNR1B, MTX1, MTX2, MUC1, MUC16, MUC20, MUC4, MUSK, MYH9, MYO16, MYO1A, MYO1C, MYO6, MYO7A, NAGPA, NAPSB, NARF, NBEA, NCAM1, NCAM2, NCF4, NCKAP1L, NCR2, NCR3, NCSTN, ND1, NDST2, NDUFA1, NDUFA13, NDUFA2, NDUFA6, NDUFA9, NDUFAB1, NDUFB6, NDUFS1, NDUFS2, NDUFS3, NDUFS4, NDUFS7, NDUFS8, NDUFV1, NECAB3, NECAP2, NEO1, NEU3, NEU4, NEXN, NF2, NFAM1, NGFR, NINJ2, NKG7, NLGN1, NMBR, NMUR1, NNT, NOD2, NOTCH2, NOTCH3, NOTCH4, NOX4, NPC1, NPFFR2, NPHS1, NPHS2, NPLOC4, NPR2, NPTN, NPY1R, NPY2R, NPY5R, NPY6R, NRAP, NRBP1, NRM, NRSN1, NRSN2, NRXN1, NRXN3, NTNG1, NTNG2, NTRK1, NTRK2, NTRK3, NTSR1, NTSR2, NUCB2, NUMB, NUP107, NUP133, NUP153, NUP160, NUP214, NUP50, NUP54, NUP62, NUP85, NUP88, NUP98, NUTF2, NXT1, OAS2, OCA2, OCLN, OGDH, OLR1, OPA1, OPCML, OPN1LW, OPN1MW, OPN1SW, OPN3, OPN4, OPRD1, OPRK1, OPRL1, OPRM1, OPRS1, OR10J1, OR1D2, OR1D4, OR1E2, OR3A2, OR52A1, ORAI1, ORMDL1, OSMR, OSTalpha, OSTbeta, OTOF, OXA1L, OXGR1, OXTR, P11, P2RX1, P2RX3, P2RX4, P2RX5, P2RX7, P2RXL1, P2RY1, P2RY11, P2RY2, P2RY4, P2RY6, PAG1, PAK1, PALM, PAMCI, PARD3, PARD6A, PARD6B, PARP1, PCDH1, PCDH11X, PCDH12, PCDH24, PCDH7, PCDH8, PCDHA1, PCDHA10, PCDHA11, PCDHA2, PCDHA3, PCDHA4, PCDHA5, PCDHA6, PCDHA7, PCDHA8, PCDHAC1, PCDHAC2, PCDHB11, PCDHB12, PCDHB15, PCDHB17, PCDHB2, PCDHB3, PCDHB4, PCDHB6, PCSK7, PDGFRA, PDPK1, PDPN, PDYN, PDZD2, PDZD3, PECAM1, PEX1, PEX11A, PEX12, PEX13, PEX14, PEX16, PEX19, PEX3, PGM5, PGRMC1, PGRMC2, PHB, PHB2, PHEX, PI4K2A, PICALM, PICK1, PIGA, PIGB, PIGC, PIGF, PIGK, PIGO, PIGR, PIGS, PIGT, PIGU, PIGV, PIGY, PIP5K3, PITPNM3, PKD1, PKD1L1, PKD2, PKD2L2, PKDREJ, PKHD1, PKP2, PKP4, PLA2G4C, PLA2R1, PLAUR, PLCE1, PLD1, PLD2, PLEKHB1, PLP2, PLSCR1, PLXNA3, PMPCA, PNN, PNPLA3, PNPLA8, PODXL, PODXL2, POLA1, POMT1, PPAP2A, PPAP2C, PPOX, PPP2R5C, PPT1, PPYR1, PRAF2, PREX1, PRICKLE1, PRKAR2A, PRKCB1, PRKCZ, PRKD1, PRLHR, PRMT8, PRNP, PROCR, PROM1, PRPH2, PRRG1, PRRG2, PRSS21, PRSS8, PSAP, PSCA, PSCD1, PSCD2, PSCD3, PSEN1, PSEN2, PSENEN, PSMG1, PTAFR, PTCH1, PTCH2, PTGER1, PTGER2, PTGER3, PTGER4, PTGFR, PTGIR, PTH2R, PTHR1, PTK7, PTPN5, PTPN6, PTPRA, PTPRB, PTPRC, PTPRCAP, PTPRD, PTPRF, PTPRG, PTPRH, PTPRJ, PTPRK, PTPRM, PTPRN, PTPRN2, PTPRO, PTPRS, PTPRT, PTPRU, PTPRZ1, PTRF, PVR, PVRL1, PVRL2, PVRL3, PXMP3, PXMP4, QSOX1, RAB11FIP5, RAB13, RAB14, RAB22A, RAB26, RAB35, RAB5B, RAC3, RAE1, RAF1, RALBP1, RAMP1, RAMP2, RAMP3, RANBP2, RANBP5, RANGAP1, RAPGEF6, RARRES1, RASA3, RASGRP4, RCE1, RDH8, RER1, RETSAT, RGMB, RGR, RGS1, RGS19, RHAG, RHBDL1, RHBG, RHCE, RHCG, RHD, RHO, RHOB, RHOT1, RHOT2, RIC8B, RIMS1, RIN1, RIT1, RIT2, RND1, RNF139, RNF144B, RNPEP, ROBO1, ROBO4, ROM1, ROR1, ROR2, ROS1, RP1-21018.1, RP5-886K2.1, RPN1, RPN2, RRH, RTN1, RTN4, RTN4RL1, RTN4RL2, RXFP3, RYK, RYR1, RYR2, SBF1, SBF2, SC4MOL, SCARB1, SCARB2, SCARF1, SCN2B, SCN3B, SCN4B, SCN8A, SCNN1G, SCRN1, SCTR, SCUBE1, SDC1, SDCBP, SDHA, SDHC, SDHD, SEC14L1, SEC22A, SEC22C, SEC23B, SEC24B, SECTM1, SELE, SELL, SELP, SELPLG, SELS, SEMA4F, SENP2, 5-Sep, SERINC1, SERINC3, SERPINA5, SGCA, SGCB, SGCD, SGCE, SGCG, SGMS1, SGMS2, SH2B2, SHC1, SHH, SHROOM2, SHROOM3, SIGIRR, SIGLEC6, SIGLEC7, SIGLEC8, SILV, SIRPA, SIRPB1, SIRPG, SIT1, SKAP2, SLA2, SLC10A1, SLC10A2, SLC11A1, SLC11A2, SLC12A1, SLC12A2, SLC12A3, SLC12A4, SLC12A6, SLC12A7, SLC12A9, SLC13A2, SLC13A4, SLC14A1, SLC14A2, SLC15A1, SLC15A2, SLC16A1, SLC16A10, SLC16A2, SLC16A3, SLC16A4, SLC16A5, SLC16A6, SLC16A7, SLC16A8, SLC17A1, SLC17A2, SLC17A3, SLC17A4, SLC17A5, SLC17A7, SLC18A2, SLC18A3, SLC19A1, SLC1A1, SLC1A4, SLC1A5, SLC1A6, SLC20A1, SLC20A2, SLC22A1, SLC22A11, SLC22A12, SLC22A13, SLC22A14, SLC22A16, SLC22A18, SLC22A2, SLC22A3, SLC22A4, SLC22A5, SLC22A6, SLC22A7, SLC22A8, SLC23A1, SLC23A2, SLC24A1, SLC25A1, SLC25A11, SLC25A13, SLC25A14, SLC25A15, SLC25A17, SLC25A22, SLC25A3, SLC25A4, SLC25A5, SLC25A6, SLC26A1, SLC26A2, SLC26A4, SLC28A1, SLC28A2, SLC29A1, SLC29A2, SLC2A2, SLC2A4, SLC2A5, SLC2A8, SLC30A3, SLC30A5, SLC31A1, SLC31A2, SLC33A1, SLC34A1, SLC34A2, SLC34A3, SLC35A1, SLC35B2, SLC38A3, SLC39A1, SLC39A2, SLC3A1, SLC40A1, SLC43A1, SLC44A1, SLC44A2, SLC46A1, SLC4A1, SLC4A11, SLC4A3, SLC4A4, SLC5A1, SLC5A2, SLC5A3, SLC5A5, SLC5A6, SLC5A7, SLC6A2, SLC6A20, SLC6A3, SLC6A4, SLC6A7, SLC6A9, SLC7A1, SLC7A10, SLC7A11, SLC7A2, SLC7A4, SLC7A6, SLC7A7, SLC7A8, SLC7A9, SLC8A1, SLC9A1, SLC9A2, SLC9A3R2, SLC9A5, SLC9A6, SLC9A7, SLCO1B1, SLCO1B3, SLCO2A1, SLMAP, SLN, SMO, SMPD2, SNAP23, SNAP29, SNIP, SNTB1, SNTB2, SNTG1, SNTG2, SNUPN, SOAT1, SOD1, SORBS1, SORBS3, SORCS1, SORL1, SP140, SPAG9, SPAM1, SPAR, SPCS1, SPINK5, SPINT2, SPN, SREBF1, SRP9, SRPR, SRPX, SSH1, SSPN, SSR1, SSR2, SSTR1, SSTR2, SSTR3, SSTR4, SSTR5, ST14, ST3GAL5, ST3GAL6, ST6GALNAC6, ST8SIA1, STAB1, STAB2, STBD1, STEAP1, STEAP2, STEAP4, STIM1, STOM, STS, STT3A, STT3B, STX10, STX12, STX16, STX1B, STX2, STX3, STX4, STX5, STX6, STX8, STYK1, SUMO1, SURF1, SVIL, SYK, SYNE1, SYNGR1, SYNGR2, SYNGR3, SYPL1, SYTL1, SYTL2, SYTL4, TAAR5, TACR1, TACR2, TACR3, TACSTD1, TACSTD2, TAPBP, TAPT1, TAS2R16, TBC1D8, TBXA2R, TCIRG1, TDGF1, TEGT, TEK, TETRAN, TFRC, TGFBR1, TGFBR2, TGFBR3, TGFBRAP1, TGM1, TGM3, THBD, THY1, TIE1, TIMM10, TIMM13, TIMM17A, TIMM17B, TIMM23, TIMM50, TIMM8B, TIMM9, TJP1, TLOC1, TLR1, TLR10, TLR2, TLR3, TLR4, TLR6, TM4SF1, TM4SF4, TM4SF5, TM7SF2, TM9SF1, TM9SF2, TMED10, TMED2, TMEM1, TMEM11, TMEM16G, TMEM2, TMEM29, TMEM47, TMEM48, TMEM5, TMEM50B, TMEM59L, TMPO, TMPRSS11D, TMPRSS11E, TMPRSS2, TMPRSS3, TMPRSS6, TNFRSF10C, TNFRSF13B, TNFRSF17, TNFRSF1A, TNFRSF25, TNFRSF4, TNFRSF9, TNFSF10, TNFSF12, TNFSF15, TNFSF4, TNFSF8, TNK1, TOM1, TOMM22, TOMM34, TPBG, TPO, TPP1, TPR, TPSG1, TPTE, TRAK2, TRAM1, TRAT1, TRDN, TREH, TRHDE, TRHR, TRIM23, TRIM27, TRIP6, TRO, TRPA1, TRPC1, TRPC3, TRPC4, TRPC5, TRPC6, TRPM1, TRPM2, TRPM5, TRPV1, TRPV2, TRPV4, TRPV5, TRPV6, TSHR, TSPAN1, TSPAN12, TSPAN13, TSPAN15, TSPAN16, TSPAN2, TSPAN3, TSPAN31, TSPAN4, TSPAN7, TSPAN9, TTYH1, TUSC3, TXNDC1, TXNDC4, TYRO3, TYROBP, UBIAD1, UBQLN4, UCP3, UMOD, UNC93A, UPK1A, UPK2, UQCRB, UQCRC1, UQCRH, USO1, UST, UTRN, VAMP1, VAMP2, VAMP3, VAMP4, VAMP5, VANGL2, VAPA, VAPB, VAT1, VCL, VDAC1, VDAC2, VDAC3, VIPR1, VIPR2, VLDLR, VSIG2, VTI1A, WFS1, WNK4, WTAP, XCR1, XK, XPO1, XPO7, XPR1, YKT6, ZAP70 and ZYX.

Among them, the live cell marker protein is preferably one selected from the group consisting of A4GNT, AAAS, AADAC, ABCA1, ABCA2, ABCA3, ABCA7, ABCB1, ABCB11, ABCB4, ABCB7, ABCB8, ABCC1, ABCC2, ABCC3, ABCC4, ABCC5, ABCC6, ABCD2, ABCD3, ABCD4, ABCF2, ABCG1, ABCG2, ABRA, ACCN1, ACCN2, ACCN3, ACCN4, ACE, ACP2, ACP5, ACTL6A, ACTN1, ACTN2, ACTN3, ACVR1, ACVR1B, ACVR1C, ACVR2A, ACVR2B, ACVRL1, ADAM11, ADAM12, ADAM17, ADAM2, ADAM23, ADAM29, ADAM30, ADAM8, ADAM9, ADCY3, ADCY7, ADCY8, ADCY9, ADI1, ADORA1, ADORA2A, ADORA2B, ADORA3, ADRA1A, ADRA1B, ADRA1D, ADRA2A, ADRA2B, ADRA2C, ADRB1, ADRB2, ADRB3, ADRM1, AFTPH, AGER, AGPAT3, AGTR1, AGTR2, AGTRL1, AIFM2, AIFM3, AKAP7, AKT1, AKTIP, ALAS2, ALG3, ALK, ALOX12, AMBP, AMFR, AMTN, ANKFY1, ANKH, ANKRA2, ANKRD20A1, ANPEP, ANXA2, AOC3, AP1G2, AP1GBP1, AP1S1, AP2S1, AP3S1, APC2, APH1A, APH1B, APOM, APP, AQP1, AQP2, AQP4, AQP5, AQP6, AQP7, AQP8, AQP9, ARCN1, ARF1, ARF6, ARFGEF2, ARFIP1, ARFIP2, ARHGEF1, ARL6, ARL6IP5, ARRB1, ARRB2, ART1, ART3, ASGR1, ASPH, ATF6, ATP12A, ATP1A1, ATP1A2, ATP1A3, ATP1A4, ATP1B1, ATP1B3, ATP1B4, ATP2A2, ATP2B1, ATP2B3, ATP2B4, ATP2C1, ATP4A, ATP5A1, ATP5B, ATP5C1, ATP5D, ATP5E, ATP5G1, ATP5G2, ATP5G3, ATP5J, ATP5O, ATP6AP1, ATP6AP2, ATP6V0A4, ATP6V0B, ATP6V0C, ATP6V1A, ATP6V1B1, ATP6V1C1, ATP6V1E1, ATP6V1F, ATP7A, ATP8B1, ATRN, AVPR1A, AVPR1B, AVPR2, AXIN1, AXL, B3GAT1, B3GNT3, B4GALT1, B4GALT4, B4GALT7, BACE1, BAII, BAI2, BAI3, BAIAP2, BAK1, BAMBI, BASP1, BAX, BCAM, BCAP31, BCAR1, BCL10, BCL2, BCL2L13, BCS1L, BDKRB1, BDKRB2, BEST1, BET1, BET1L, BFAR, BMPR1A, BMPR2, BNIP1, BNIP2, BNIP3, BNIP3L, BPI, BRS3, BSCL2, BSND, BST1, BST2, BTK, BTN1A1, BTN2A1, C11orf2, C1GALT1, C1QBP, C3AR1, C3orf31, C5AR1, C6orf25, C7, C8A, C8B, C9, C9orf127, C9orf58, CA12, CA14, CA9, CACNA1A, CACNA1B, CACNA1C, CACNA1D, CACNA1E, CACNA1F, CACNA1G, CACNA1H, CACNA1S, CACNA2D1, CACNB1, CACNB2, CACNB3, CACNB4, CACNG1, CACNG2, CACNG4, CADM1, CADM3, CALCRL, CALM3, CALY, CAPRIN1, CARD11, CASK, CASP7, CASR, CAV1, CAV2, CAV3, CBL, CBX5, CCBP2, CCDC70, CCDC8, CCDC88A, CCKAR, CCKBR, CCR1, CCR10, CCR2, CCR3, CCR4, CCR5, CCR6, CCR7, CCR8, CCR9, CCRL1, CCRL2, CD14, CD151, CD160, CD163, CD164, CD19, CD1B, CD1C, CD1D, CD200, CD22, CD226, CD24, CD247, CD28, CD300C, CD33, CD36, CD37, CD38, CD3E, CD4, CD40, CD40LG, CD46, CD47, CD48, CD5, CD52, CD53, CD55, CD6, CD63, CD68, CD69, CD7, CD70, CD72, CD74, CD79A, CD79B, CD81, CD82, CD83, CD84, CD8B, CD96, CD97, CD99, CDC42, CDC42BPA, CDC42BPB, CDC42EP2, CDC42SE1, CDC42SE2, CDH1, CDH13, CDH15, CDH24, CDK5, CDK5R1CEACAM4, CEACAM5, CEACAM6, CEACAM7, CEACAM8, CELSR1, CELSR3, CENPF, CENTA1, CENTA2, CEPT1, CFTR, CHL1, CHMP1A, CHRM1, CHRM2, CHRM3, CHRM4, CHRM5, CHRNA1, CHRNA2, CHRNA3, CHRNA4, CHRNA6, CHRNA7, CHRNB2, CHRNB3, CHRNB4, CHRND, CHRNE, CHRNG, CHST7, CIB1, CKAP4, CKLF, CLCA1, CLCA2, CLCA4, CLCN1, CLCN3, CLCN5, CLCNKA, CLCNKB, CLEC10A, CLEC1A, CLEC1B, CLEC2B, CLEC2D, CLEC4A, CLEC4M, CLEC5A, CLEC7A, CLN3, CLN5, CLNS1A, CLPTM1, CMKLR1, CNGA1, CNIH2, CNKSR1, CNN2, CNPY2, CNR1, CNR2, CNTFR, CNTN2, COG2, COL13A1, COL17A1, COLEC12, COPA, COPB1, COPB2, COPE, COPG, COPG2, COPZ1, CORIN, CORO1A, COX15, COX18, COX6B2, CPE, CPM, CR1, CRHR1, CRHR2, CRIM1, CRIPAK, CRNN, CRTAM, CSF1R, CSF2RA, CSF2RB, CSF3R, CSK, CSNK2A1, CSPG4, CSPG5, CTGF, CTLA4, CTNNA3, CTNS, CUBN, CUTA, CUZD1, CX3CL1, CX3CR1, CXCR3, CXCR4, CXCR5, CXCR6, CYB561, CYB5R2, CYB5R3, CYBB, CYSLTR1, DAB2, DAD1, DAG1, DARC, DC2, DCBLD2, DCLK1, DCT, DDOST, DDR1, DDR2, DDX19B, DEGS1, DERL1, DERL2, DERL3, DGKD, DGKK, DHCR7, DHRS9, DKK1, DLG2, DLG4, DLK1, DMBT1, DMD, DNER, DNM2, DOLPP1, DOPEY1, DOPEY2, DPM1, DPM2, DPM3, DRD1, DRD2, DRD3, DRD4, DRD5, DSC1, DSC3, DSCAM, DST, DTNBP1, DULLARD, DYSF, EBI2, EBI3, EBP, ECE1, ECE2, ECEL1, EDA, EDEM1, EDG1, EDG2, EDG3, EDG4, EDG6, EDG7, EDNRA, EDNRB, EEA1, EFNA1, EFNA3, EFNA4, EFNA5, EFNB1, EFNB2, EFNB3, EGF, EGFR, EIF2B1, EIF5A, ELMO1, ELTD1, EMCN, EMD, EMP1, EMP2, EMR1, EMR2, EMR3, EMR4, ENG, ENOX2, ENPEP, ENPP1, ENPP2, ENPP3, ENTPD1, ENTPD4, EPB41, EPB41L2, EPB41L3, EPB42, EPC1, EPGN, EPHA1, EPHA3, EPHA4, EPHA5, EPHA8, EPHB1, EPHB2, EPHB3, EPHB4, EPOR, EPS15, ERBB2IP, ERBB3, ERBB4, ERC1, ERC2, EREG, ERLIN1, ERLIN2, ERMAP, ERN1, EVI2B, EVL, EXT1, EXT2, EZR, F2R, F2RL1, F2RL2, F2RL3, F3, FADS2, FAM84B, FASLG, FAT, FCAR, FCER1A, FCER1G, FCER2, FCGR3A, FDFT1, FER1L3, FFAR1, FFAR2, FFAR3, FGFR1, FGFR3, FGFR4, FIS1, FLOT1, FLT1, FLT3, FLT4, FLVCR1, FOLH1, FOLR1, FOLR3, FPR1, FPRL1, FPRL2, FRAG1, FRAP1, FRS2, FRZB, FSHR, FUT1, FUT7, FXYD1, FXYD3, FXYD5, FXYD6, FYN, FZD10, FZD2, FZD3, FZD4, FZD6, FZD7, FZD8, FZD9, GABARAP, GABBR1, GABBR2, GABRA1, GABRA2, GABRA3, GABRA4, GABRA5, GABRA6, GABRB1, GABRB2, GABRB3, GABRD, GABRE, GABRG2, GABRQ, GABRR1, GABRR2, GAL3ST1, GAL3ST2, GALNT3, GALR1, GALR2, GALR3, GAS1, GBA2, GBAS, GCA, GCGR, GCNT1, GFRA2, GFRA3, GGCX, GGTLA1, GHR, GHRHR, GHSR, GIPC1, GIPR, GJA4, GJA8, GJB1, GJB2, GJD3, GLE1, GLG1, GLP1R, GLP2R, GLRA1, GML, GNA11, GNA14, GNA15, GNAI1, GNAQ, GNAS, GNAZ, GNG4, GNRHR, GOLGA3, GOLGA5, GOLGB1, GOLIM4, GOLM1, GOSR1, GOT2, GP1BA, GP5, GP6, GP9, GPA33, GPAA1, GPC1, GPC3, GPC4, GPC5, GPC6, GPER, GPNMB, GPR1, GPR109B, GPR110, GPR111, GPR112, GPR113, GPR114, GPR115, GPR116, GPR12, GPR123, GPR124, GPR125, GPR126, GPR128, GPR133, GPR135, GPR137B, GPR143, GPR144, GPR15, GPR17, GPR175, GPR176, GPR182, GPR19, GPR20, GPR21, GPR22, GPR23, GPR25, GPR3, GPR31, GPR32, GPR34, GPR35, GPR37, GPR39, GPR4, GPR42, GPR44, GPR45, GPR50, GPR52, GPR55, GPR56, GPR6, GPR64, GPR65, GPR68, GPR75, GPR97, GPR98, GPRC5A, GPRC5C, GRB10, GRIA1, GRIA2, GRIA3, GRIA4, GRID2, GRIK1, GRIK2, GRIK3, GRIK4, GRIN1, GRIN2A, GRIN2B, GRIN2C, GRINL1A, GRM1, GRM2, GRM3, GRM4, GRM5, GRM6, GRM7, GRM8, GRPR, GUCY2D, GUCY2F, GYPA, GYPB, GYPC, GYPE, GZMA, GZMB,HAS1, HAS2, HAS3, HBEGF, HCCS, HCN2, HCRTR1, HCRTR2, HDAC11, HDLBP, HERPUD1, HFE, HLA-DOA, HLA-DRA, HLA-DRB3, HLA-DRB4, HLA-DRB5, HMGCR, HNRPM, HOMER1, HPN, HPS1, HRAS, HRB, HRH1, HRH2, HRH3, HS3ST1, HS3ST2, HS3ST3A1, HS3ST3B1, HS6ST1, HSD17B10, HSD17B2, HSD3B1, HSD3B2, HSP90B1, HTATIP2, HTR1A, HTR1B, HTR1D, HTR1E, HTR1F, HTR2A, HTR2B, HTR3A, HTR3B, HTR4, HTR5A, HTR6, HTR7, HTRA2, HVCN1, ICA1, ICAM1, ICAM2, ICAM3, ICAM4, ICAM5, IFITM1, IFITM3, IFNAR1, IFNAR2, IFNGR1, IFNGR2, IGF2R, IGSF1, IGSF2, IGSF6, IGSF8, IL10RA, IL10RB, IL11RA, IL12RB1, IL12RB2, IL13RA1, IL15, IL17RB, IL18R1, IL1R1, IL1RAP, IL1RL2, IL27RA, IL28RA, IL29, IL2RA, IL2RB, IL2RG, IL4R, IL5RA, IL6R, IL6ST, IL8RB, IL9R, IMMT, INSR, IPO7, IRS4, ITGA1, ITGA10, ITGA11, ITGA2, ITGA3, ITGA5, ITGA8, ITGA9, ITGAD, ITGAE, ITGAM, ITGAX, ITGB1, ITGB3, ITGB4, ITGB6, ITGB7, ITGB8, ITLN1, ITPR1, ITPR2, IPR3, IZUMO1, JAG2, JTB, KCNA1, KCNA2, KCNA3, KCNA4, KCNA5, KCNA6, KCNB2, KCNC1, KCNC3, KCNC4, KCND3, KCNE1, KCNE1L, KCNE2, KCNG2, KCNG3, KCNH1, KCNH2, KCNH4, KCNJ1, KCNJ10, KCNJ11, KCNJ14, KCNJ15, KCNJ2, KCNJ3, KCNJ4, KCNJ5, KCNJ6, KCNJ8, KCNK1, KCNK3, KCNK5, KCNK6, KCNMA1, KCNMB2, KCNMB3, KCNMB4, KCNN1, KCNN3, KCNN4, KCNQ1, KCNQ2, KCNQ3, KCNQ5, KCNS1, KDELR1, KDELR2, KDR, KEL, KHDRBS1, KIF1B, KIR2DL1, KIR2DL3, KIR2DL4, KIR2DS3, KIR2DS4, KIR3DL2, KIRREL3, KISSIR, KL, KLRA1, KLRB1, KLRC1, KLRC2, KLRD1, KLRF1, KLRG1, KLRK1, KPNA1, KPNA3, KPNB1, KRT1, KRTCAP2, KTN1, L2HGDH, L3MBTL4, LAMB1, LAMC3, LAMP1, LAMP2, LAMP3, LANCL1, LANCL2, LAPTM5, LARGE, LAT, LAT2, LAX1, LAYN, LBR, LCK, LCT, LDLR, LDLRAP1, LEMD3, LETM1, LGALS3, LGALS4, LGICZ1, LGR5, LHCGR, LIFR, LILRA3, LILRB1, LILRB2, LILRB3, LIMA1, LMAN1, LMAN2L, LMNB1, LMTK2, LNPEP, LPHN1, LPHN2, LPHN3, LRIT1, LRMP, LRP1, LRP12, LRP3, LRP5, LRP6, LRPAP1, LRRC32, LRRC4C, LST1, LTB4R, LTB4R2, LTC4S, LTK, LY6E, LY75, LY86, LY96, LYN, LYPD3, LYVE1, M6PR, MADD, MAEA, MAGEA1, MAGEE1, MAGT1, MAL, MALL, MAN1A2, MAN1B1, MAN1C1, MAP1LC3A, MAP1LC3B, MAP1LC3C, MAP3K12, MAP4K2, MAPT, MARCKSL1, MARCO, MAS1, MATR3, MBTPS2, MC1R, MC2R, MC3R, MC4R, MC5R, MCHR1, MCL1, MCTP1, MCTP2, MDGA1, MEP1A, MEP1B, MERTK, MET, MFI2, MFN2, MFRP, MGAT1, MGAT3, MIP, MIR16, MLANA, MLLT4, MLNR, MMD, MME, MMP14, MMP15, MMP16, MMP17, MMP24, MPL, MPP1, MPP2, MPP3, MPV17, MPZ, MPZL1, MRAS, MRC1, MRPL19, MRPL32, MRVI1, MS4A1, MS4A2, MSN, MSR1, MST1R, MSTO1, MTFR1, MTNR1A, MTNR1B, MTX1, MTX2, MUC1, MUC16, MUC20, MUC4, MUSK, MYH9, MYO16, MYO1A, MYO1C, MYO6, MYO7A, NAGPA, NAPSB, NARF, NBEA, NCAM1, NCAM2, NCF4, NCKAP1L, NCR2, NCR3, NCSTN, ND1, NDST2, NDLTFA1, NDUFA13, NDUFA2, NDUFA6, NDUFA9, NDUFAB1, NDUFB6, NDUFS1, NDUFS2, NDUFS3, NDUFS4, NDUFS7, NDUFS8, NDUFV1, NECAB3, NECAP2, NEO1, NEU3, NEU4, NEXN, NF2, NFAM1, NGFR, NINJ2, NKG7, NLGN1, NMBR, NMUR1, NNT, NOD2, NOTCH2, NOTCH3, NOTCH4, NOX4, NPC1, NPFFR2, NPHS1, NPHS2, NPLOC4, NPR2, NPTN, NPY1R, NPY2R, NPY5R, NPY6R, NRAP, NRBP1, NRM, NRSN1, NRSN2, NRXN1, NRXN3, NTNG1, NTNG2, NTRK1, NTRK2, NTRK3, NTSR1, NTSR2, NUCB2, NUMB, NUP107, NUP133, NUP153, NUP160, NUP214, NUP50, NUP54, NUP62, NUP85, NUP88, NUP98, NUTF2, NXT1, OAS2, OCA2, OGDH, OLR1, OPA1, OPCML, OPN1LW, OPN1MW, OPN1SW, OPN3, OPN4, OPRD1, OPRK1, OPRL1, OPRM1, OPRS1, OR10J1, OR1D2, OR1D4, OR1E2, OR3A2, OR52A1, ORAI1, ORMDL1, OSMR, OSTalpha, OSTbeta, OTOF, OXA1L, OXGR1, OXTR, P11, P2RX1, P2RX3, P2RX4, P2RX5, P2RX7, P2RXL1, P2RY1, P2RY11, P2RY2, P2RY4, P2RY6, PAG1, PAK1, PALM, PAMCI, PARP1, PCDH1, PCDH11X, PCDH12, PCDH24, PCDH7, PCDH8, PCDHA1, PCDHA10, PCDHA11, PCDHA2, PCDHA3, PCDHA4, PCDHA5, PCDHA6, PCDHA7, PCDHA8, PCDHAC1, PCDHAC2, PCDHB11, PCDHB12, PCDHB15, PCDHB17, PCDHB2, PCDHB3, PCDHB4, PCDHB6, PCSK7, PDGFRA, PDPK1, PDPN, PDYN, PDZD2, PDZD3, PECAM1, PEX1, PEX11A, PEX12, PEX13, PEX14, PEX16, PEX19, PEX3, PGM5, PGRMC1, PGRMC2, PHB, PHB2, PHEX, PI4K2A, PICALM, PICK1, PIGA, PIGB, PIGC, PIGF, PIGK, PIGO, PIGR, PIGS, PIGT, PIGU, PIGV, PIGY, PIP5K3, PITPNM3, PKD1, PKD1L1, PKD2, PKD2L2, PKDREJ, PKHD1, PKP2, PKP4, PLA2G4C, PLA2R1, PLAUR, PLCE1, PLD1, PLD2, PLEKHB1, PLP2, PLSCR1, PLXNA3, PMPCA, PNN, PNPLA3, PNPLA8, PODXL, PODXL2, POLA1, POMT1, PPAP2A, PPAP2C, PPOX, PPP2R5C, PPT1, PPYR1, PRAF2, PREX1, PRICKLE1, PRKAR2A, PRKCB1, PRKD1, PRLHR, PRMT8, PRNP, PROCR, PROM1, PRPH2, PRRG1, PRRG2, PRSS21, PRSS8, PSAP, PSCA, PSCD1, PSCD2, PSCD3, PSEN1, PSEN2, PSENEN, PSMG1, PTAFR, PTCH1, PTCH2, PTGER1, PTGER2, PTGER3, PTGER4, PTGFR, PTGIR, PTH2R, PTHR1, PTK7, PTPN5, PTPN6, PTPRA, PTPRB, PTPRC, PTPRCAP, PTPRD, PTPRF, PTPRG, PTPRH, PTPRJ, PTPRK, PTPRM, PTPRN, PTPRN2, PTPRO, PTPRS, PTPRT, PTPRU, PTPRZ1, PTRF, PVR, PVRL1, PVRL2, PVRL3, PXMP3, PXMP4, QSOX1, RAB11FIP5, RAB14, RAB22A, RAB26, RAB35, RAB5B, RAC3, RAE1, RAF1, RALBP1, RAMP1, RAMP2, RAMP3, RANBP2, RANBP5, RANGAP1, RAPGEF6, RARRES1, RASA3, RASGRP4, RCE1, RDH8, RER1, RETSAT, RGMB, RGR, RGS1, RGS19, RHAG, RHBDL1, RHBG, RHCE, RHCG, RHD, RHO, RHOB, RHOT1, RHOT2, RIC8B, RIMS1, RIN1, RIT1, RIT2, RND1, RNF139, RNF144B, RNPEP, ROBO1, ROBO4, ROM1, ROR1, ROR2, ROS1, RP1-21O18.1, RP5-886K2.1, RPN1, RPN2, RRH, RTN1, RTN4, RTN4RL1, RTN4RL2, RXFP3, RYK, RYR1, RYR2, SBF1, SBF2, SC4MOL, SCARB1, SCARB2, SCARF1, SCN2B, SCN3B, SCN4B, SCN8A, SCNN1G, SCRN1, SCTR, SCUBE1, SDC1, SDCBP, SDHA, SDHC, SDHD, SEC14L1, SEC22A, SEC22C, SEC23B, SEC24B, SECTM1, SELE, SELL, SELP, SELPLG, SELS, SEMA4F, SENP2, 5-Sep, SERINC1, SERINC3, SERPINA5, SGCA, SGCB, SGCD, SGCE, SGCG, SGMS1, SGMS2, SH2B2, SHC1, SHH, SHROOM3, SIGIRR, SIGLEC6, SIGLEC7, SIGLEC8, SILV, SIRPA, SIRPB1, SIRPG, SIT1, SKAP2, SLA2, SLC10A1, SLC10A2, SLC11A1, SLC11A2, SLC12A1, SLC12A2, SLC12A3, SLC12A4, SLC12A6, SLC12A7, SLC12A9, SLC13A2, SLC13A4, SLC14A1, SLC14A2, SLC15A1, SLC15A2, SLC16A1, SLC16A10, SLC16A2, SLC16A3, SLC16A4, SLC16A5, SLC16A6, SLC16A7, SLC16A8, SLC17A1, SLC17A2, SLC17A3, SLC17A4, SLC17A5, SLC17A7, SLC18A2, SLC18A3, SLC19A1, SLC1A1, SLC1A4, SLC1A5, SLC1A6, SLC20A1, SLC20A2, SLC22A1, SLC22A11, SLC22A12, SLC22A13, SLC22A14, SLC22A16, SLC22A18, SLC22A2, SLC22A3, SLC22A4, SLC22A5, SLC22A6, SLC22A7, SLC22A8, SLC23A1, SLC23A2, SLC24A1, SLC25A1, SLC25A11, SLC25A13, SLC25A14, SLC25A15, SLC25A17, SLC25A22, SLC25A3, SLC25A4, SLC25A5, SLC25A6, SLC26A1, SLC26A2, SLC26A4, SLC28A1, SLC28A2, SLC29A1, SLC29A2, SLC2A2, SLC2A4, SLC2A5, SLC2A8, SLC30A3, SLC30A5, SLC31A1, SLC31A2, SLC33A1, SLC34A1, SLC34A2, SLC34A3, SLC35A1, SLC35B2, SLC38A3, SLC39A1, SLC39A2, SLC3A1, SLC40A1, SLC43A1, SLC44A1, SLC44A2, SLC46A1, SLC4A1, SLC4A11, SLC4A3, SLC4A4, SLCSA1, SLC5A2, SLC5A3, SLC5A5, SLC5A6, SLC5A7, SLC6A2, SLC6A20, SLC6A3, SLC6A4, SLC6A7, SLC6A9, SLC7A1, SLC7A10, SLC7A11, SLC7A2, SLC7A4, SLC7A6, SLC7A7, SLC7A8, SLC7A9, SLC8A1, SLC9A1, SLC9A2, SLC9A3R2, SLC9A5, SLC9A6, SLC9A7, SLCO1B1, SLCO1B3, SLCO2A1, SLMAP, SLN, SMO, SMPD2, SNAP23, SNAP29, SNIP, SNTB1, SNTB2, SNTG1, SNTG2, SNUPN, SOAT1, SOD1, SORBS1, SORBS3, SORCS1, SORL1, SP140, SPAG9, SPAM1, SPAR, SPCS1, SPINK5, SPINT2, SPN, SREBF1, SRP9, SRPR, SRPX, SSH1, SSPN, SSR1, SSR2, SSTR1, SSTR2, SSTR3, SSTR4, SSTR5, ST14, ST3GAL5, ST3GAL6, ST6GALNAC6, ST8SIA1, STAB1, STAB2, STBD1, STEAP1, STEAP2, STEAP4, STIM1, STOM, STS, STT3A, STT3B, STX10, STX12, STX16, STX1B, STX2, STX3, STX4, STX5, STX6, STX8, STYK1, SUMO1, SURF1, SVIL, SYK, SYNE1, SYNGR1, SYNGR2, SYNGR3, SYPL1, SYTL1, SYTL2, SYTL4, TAAR5, TACR1, TACR2, TACR3, TACSTD1, TACSTD2, TAPBP, TAPT1, TAS2R16, TBC1D8, TBXA2R, TCIRG1, TDGF1, TEGT, TEK, TETRAN, TFRC, TGFBR2, TGFBR3, TGFBRAP1, TGM1, TGM3, THBD, THY1, TIE1, TIMM10, TIMM13, TIMM17A, TIMM17B, TIMM23, TIMM50, TIMM8B, TIMM9, TLOC1, TLR1, TLR10, TLR2, TLR3, TLR4, TLR6, TM4SF1, TM4SF4, TM4SF5, TM7SF2, TM9SF1, TM9SF2, TMED10, TMED2, TMEM1, TMEM11, TMEM16G, TMEM2, TMEM29, TMEM47, TMEM48, TMEM5, TMEM50B, TMEM59L, TMPO, TMPRSS11D, TMPRSS11E, TMPRSS2, TMPRSS3, TMPRSS6, TNFRSF10C, TNFRSF13B, TNFRSF17, TNFRSF1A, TNFRSF25, TNFRSF4, TNFRSF9, TNFSF10, TNFSF12, TNFSF15, TNFSF4, TNFSF8, TNK1, TOM1, TOMM22, TOMM34, TPBG, TPO, TPP1, TPR, TPSG1, TPTE, TRAK2, TRAM1, TRAT1, TRDN, TREH, TRHDE, TRHR, TRIM23, TRIM27, TRIP6, TRO, TRPA1, TRPC1, TRPC3, TRPC4, TRPC5, TRPC6, TRPM1, TRPM2, TRPM5, TRPV1, TRPV2, TRPV4, TRPV5, TRPV6, TSHR, TSPAN1, TSPAN12, TSPAN13, TSPAN15, TSPAN16, TSPAN2, TSPAN3, TSPAN31, TSPAN4, TSPAN7, TSPAN9, TTYH1, TUSC3, TXNDC1, TXNDC4, TYRO3, TYROBP, UBIAD1, UBQLN4, UCP3, UMOD, UNC93A, UPK1A, UPK2, UQCRB, UQCRC1, UQCRH, USO1, UST, UTRN, VAMP1, VAMP2, VAMP3, VAMP4, VAMP5, VANGL2, VAPB, VAT1, VCL, VDAC1, VDAC2, VDAC3, VIPR1, VIPR2, VLDLR, VSIG2, VTI1A, WFS1, WTAP, XCR1, XK, XPO1, XPO7, XPR1, YKT6, ZAP70 and ZYX.

Further, among them, the live cell marker protein is more preferably a serine protease such as tripeptidyl peptidase 1 (hereinafter, also referred to as TPP1), and even more preferably TPP1 since it can be easily detected using a commercially available kit.

Examples of stromal cells used to knock out a gene encoding a live cell marker protein include endothelial cells, fibroblasts, neuronal cells, dendritic cells, macrophages, mast cells, epithelial cells, myocardial cells, hepatic cells, pancreatic islet cells, tissue stem cells and smooth muscle cells. The type of stromal cells contained in the cell structure may be of one type, or may be of two or more types. The cell type of stromal cells contained in the cell structure is not specifically limited, and can be suitably selected in consideration of the origin and type of cancer cells, the type of anticancer drug used for assessment, the *in vivo* environment in which the target anticancer activity is exhibited, etc.

A blood vessel network structure and a lymphatic network structure are important for the growth and activity of cancer cells. Therefore, the cell structure preferably includes a vascular network structure. That is, the cell structure is preferably one in which at least one of the vascular network structures of lymph vessels and blood vessels is three-dimensionally formed inside a laminate of non-vascularized cells, producing tissues closer to those *in vivo.* The vascular network structure may be formed only inside the cell structure, or may be formed so that at least part of the vascular network structure is exposed to the surface or the bottom of the cell structure.

In the present invention and the present specification, the term "vascular network structure" refers to a network structure, such as a blood vessel network or a lymphatic network, in living tissues.

A vascular network structure can be formed by incorporating vascularized endothelial cells as stromal cells. The endothelial cells contained in the cell structure may be vascular endothelial cells or lymphatic endothelial cells. Moreover, both vascular endothelial cells and lymphatic endothelial cells may be contained.

When the cell structure has a vascular network structure, the cells other than the endothelial cells in the cell structure are not specifically limited as long as they do not inhibit the endothelial cells from forming a vascular network. However, the cells are preferably cells that constitute surrounding tissues of vessels in a living body since the endothelial cells can easily form a vessel network maintaining the original function and shape. Furthermore, in order to mimic the *in vivo* cancer microenvironment, the cells other than the endothelial cells are more preferably cells containing at least fibroblasts, and even more preferably cells containing vascular endothelial cells and fibroblasts, cells containing lymphatic endothelial cells and fibroblasts, or cells containing vascular endothelial cells, lymphatic endothelial cells and fibroblasts. Further, the cells other than the endothelial cells contained in the cell structure may be derived from the same species as that of the endothelial cells or from different species. Moreover, the type of the cells other than the endothelial cells contained in the cell structure may be of one type, or may be of two or more types.

The number of endothelial cells in the cell structure is not specifically limited as long as it is sufficient to form a vascular network structure, and can be determined as appropriate in consideration of the size of the cell structure, the type of endothelial cells or cells other than endothelial cells, and the like. For example, a cell structure having a vascular network structure can be prepared by setting the abundance ratio (cell number ratio) of the endothelial cells to all the cells constituting the cell structure to 0.1% or greater. When fibroblasts are used as the cells other than endothelial cells, the number of endothelial cells in the cell structure according to the present embodiment is preferably 0.1% or greater, more preferably 0.1% or greater and 10.0% or less, and even more preferably 0.1% or greater and 5.0% or less, of the number of fibroblasts. When both vascular endothelial cells and lymphatic endothelial cells are contained as the endothelial cells, the total number of vascular endothelial cells and lymphatic endothelial cells is preferably 0.1% or greater, more preferably 0.1% or greater and 10.0% or less, and even more preferably 0.1% or greater and 5.0% or less, of the number of fibroblasts.

### (Cancer Cells)

The cancer cells may be established culture cells, or cancer cells collected from a cancer patient. The cancer cells collected from a cancer patient may be grown by culture in advance. Specifically, examples of the cancer cells include primary cancer cells collected from a cancer patient, artificially cultured cancer cells, iPS cancer stem cells, cancer stem cells, and established cancer cells prepared in advance for use in studies of cancer therapy or development of anticancer drugs. Moreover, the cancer cells may be derived from a human, or may be derived from an animal other than a human. When the cancer cells are collected from a cancer patient, cells other than the cancer cells collected from the cancer patient may be contained together with the cancer cells. Examples of the cells other than cancer cells include one or more types of cells contained in solid tissue removed after surgery.

Cancer cells are cells that are derived from somatic cells and that acquire infinite proliferation potential. Non-limiting examples of the cancer from which cancer cells are derived include breast cancer (e.g., invasive ductal carcinoma, ductal carcinoma in situ, and inflammatory breast cancer), prostate cancer (e.g., hormone-dependent prostate cancer and hormone-independent prostate cancer), pancreatic cancer (e.g., pancreatic duct cancer), gastric cancer (e.g., papillary adenocarcinoma, mucinous adenocarcinoma, and adenosquamous carcinoma), lung cancer (e.g., non-small-cell lung cancer, small-cell lung cancer, and malignant mesothelioma), colon cancer (e.g., gastrointestinal stromal tumor), rectal cancer (e.g., gastrointestinal stromal tumor), colorectal cancer (e.g., familial colorectal cancer, hereditary nonpolyposis colorectal cancer, and gastrointestinal stromal tumor), small intestinal cancer (e.g., non-Hodgkin's lymphoma and gastrointestinal stromal tumor), esophageal cancer, duodenal cancer, tongue cancer, pharyngeal cancer (e.g., nasopharyngeal cancer, oropharyngeal cancer, and hypopharyngeal cancer), head and neck cancer, salivary gland cancer, brain tumor (e.g., pineal astrocytoma, pilocytic astrocytoma, diffuse astrocytoma, and anaplastic astrocytoma), neurilemmoma, liver cancer (e.g., primary liver cancer and extrahepatic bile duct cancer), renal cancer (e.g., renal cell cancer and transitional cell cancer of the renal pelvis and ureter), gallbladder cancer, bile duct cancer, pancreatic cancer, hepatoma, endometrial cancer, cervical cancer, ovarian cancer (e.g., epithelial ovarian cancer, extragonadal germ cell tumor, ovarian germ cell tumor, and ovarian low-malignant potential tumor), bladder cancer, urethral cancer, skin cancer (e.g., intraocular (ocular) melanoma and Merkel cell carcinoma), hemangioma, malignant lymphoma (e.g., reticulosarcoma, lymphosarcoma, and Hodgkin's disease), melanoma (malignant melanoma), thyroid cancer (e.g., medullary thyroid cancer), parathyroid cancer, nasal cancer, paranasal cancer, bone tumor (e.g., osteosarcoma, Ewing's tumor, uterine sarcoma, and soft-tissue sarcoma), metastatic medulloblastoma, hemangiofibroma, dermatofibrosarcoma protuberans, retinal sarcoma, penile cancer, testicular tumor, pediatric solid cancer (e.g., Wilms tumor and pediatric renal tumor), Kaposi sarcoma, Kaposi sarcoma caused by AIDS, tumor of the maxillary sinus, fibrous histiocytoma, leiomyosarcoma, rhabdomyosarcoma, chronic myeloproliferative disorders, leukemia (e.g., acute myelogenous leukemia and acute lymphoblastic leukemia), and the like.

The type of cells contained in the cell structure, such as stromal cells and cancer cells, is not specifically limited, and may be cells collected from an animal, cells obtained by culturing cells obtained from an animal, cells obtained by subjecting cells collected from an animal to various treatments, or cultured cell lines. In the case of cells collected from animals, the sampling site is not specifically limited, and the cells may be somatic cells derived from bone, muscle, viscus, nerve, brain, skin, blood, etc., reproductive cells, or embryonic stem cells (ES cells).

Moreover, the organism species from which the cells contained in the cell structure, such as stromal cells and cancer cells, are derived is not specifically limited. For example, usable cells can be derived from humans or animals, such as monkeys, dogs, cats, rabbits, pigs, cows, mice, and rats. The cells obtained by culturing cells collected from animals may be primary cultured cells or subcultured cells. Further, the cells obtained by applying various treatments may include induced pluripotent stem cells (iPS cells) or cells after differentiation induction. The cell structure may be composed of only cells derived from the same organism species, or cells derived from several types of organism species.

The number of cancer cells in the cell structure is not specifically limited. However, when a cell structure containing endothelial cells as stromal cells is used, the ratio of the number of endothelial cells to the number of cancer cells ([number of endothelial cells]/[number of cancer cells]) in the cell structure is preferably greater than 0 and 1.5 or less in order to further mimic the *in vivo* cancer microenvironment. Moreover, when a cell structure containing endothelial cells, fibroblasts, and cancer cells is used, the ratio of the number of fibroblasts to the number of cancer cells ([number of fibroblasts]/[number of cancer cells]) in the cell structure is preferably 0.6 or greater and 100 or less, and more preferably 50 or greater and 100 or less.

### (Properties of Cell Structure)

The size and shape of the cell structure are not specifically limited. Since it is possible to form a vascular network structure in a state closer to vessels in the *in vivo* tissue and to obtain more highly accurate assessment, the cell structure preferably has a thickness of 5 µm or greater, more preferably 10 µm or greater, even more preferably 50 µm or greater, and still even more preferably 100 µm or greater. The thickness of the cell structure is preferably 500 µm or less, more preferably 400 µm or less, and even more preferably 300 µm or less. The upper and lower limits of the thickness of the cell structure can be combined as appropriate. For example, the thickness of the cell structure is preferably 5 µm or greater and 500 µm or less, more preferably 10 µm or greater and 400 µm or less, even more preferably 50 µm or greater and 300 µm or less, and still even more preferably 100 µm or greater and 300 µm or less. The number of cell layers in the cell structure is preferably about 2 or greater and 60 or less, more preferably about 5 or greater and 60 or less, and even more preferably about 10 or greater and 60 or less.

In the present invention and the present specification, the number of cell layers constituting the cell structure is measured by dividing the total number of cells constituting a three-dimensional structure by the number of cells per layer (in other words, the number of cells necessary to form one layer). The number of cells per layer can be examined in such a manner that cells are cultured in advance on a plane so that they are confluent in a cell vessel that is used in the production of a cell structure. Specifically, the number of cell layers in a cell structure formed in a certain cell vessel can be calculated by counting the total number of cells constituting the cell structure, and dividing the total number of cells by the number of cells per layer in the cell vessel.

### (Method of Producing Cell Structure)

In general, the cell structure is produced in a cell culture vessel. The cell culture vessel is not specifically limited as long as it enables production of a cell structure and enables culture of the produced cell structure. Specifically, examples of the cell culture vessel include dishes, cell culture inserts (e.g., Transwell (registered trademark) inserts, Netwell (registered trademark) inserts, Falcon (registered trademark) cell culture inserts, and Millicell (registered trademark) cell culture inserts), tubes, flasks, bottles, plates, and the like. In production of a cell structure, dishes or various cell culture inserts are preferred since they allow for more appropriate assessment using the cell structure.

The cell structure may be any structure formed from multiple cell layers containing stromal cells, and the method of producing the cell structure is not specifically limited. For example, the cell structure may be produced by laminating each layer in sequence, or forming two or more cell layers at once, or forming multiple cell layers by suitably combining both production methods. Moreover, the cell structure may be a multilayer structure in which the type of cells constituting each layer differs from layer to layer, or the type of cells constituting each layer is the same in all the layers of the structure. For example, the cell structure may be produced by forming each layer for each type of cells and laminating these cell layers in sequence, or may be produced by preparing, in advance, a cell mixture solution containing a mixture of several types of cells and forming a multilayer cell structure at once from the cell mixture solution.

Examples of the method of producing a cell structure by laminating each layer in sequence include the method disclosed in Reference 1 (JP 4919464 B), that is, alternately repeating a step of forming a cell layer and a step of bringing the formed cell layer into contact with a solution containing an extracellular matrix (ECM) component to thereby continuously laminate the cell layers. For example, the above method can be performed by preparing, in advance, a cell mixture containing all cells constituting a cell structure, and then forming each cell layer from this cell mixture so that a cell structure can be produced in which a vascular network structure is formed in the entire structure and cancer cells are scattered in the entire structure. Furthermore, by forming each cell layer for each cell type, a cell structure can be produced in which a vascular network structure is formed only in a layer formed from endothelial cells and cancer cells are present only in a specific layer.

Examples of the method of producing a cell structure by forming two or more cell layers at once include the method disclosed in Reference 2 (JP 5850419 B), that is, coating the entire cell surface in advance with a polymer containing an arginine-glycine-aspartic acid (RGD) sequence bound to integrin and a polymer interacting with the polymer containing the RGD sequence, accommodating the cells coated with an adhesive film in a cell culture vessel, and integrating the coated cells by centrifugation or the like to thereby produce a cell structure having multiple cell layers.

For example, the above method can be performed using coated cells formed by preparing, in advance, a cell mixture containing all cells constituting a cell structure, and adding an adhesive component to the cell mixture. This enables production of a cell structure in which cancer cells are scattered in the entire structure by one centrifugation. Similarly, by using coated cells formed by preparing, in advance, a cell mixture containing all cells constituting a cell structure, and adding an adhesive component to the cell mixture, a cell structure in which a vascular network structure is formed in the entire structure can be produced by one centrifugation. Moreover, it is also possible to, for example, separately prepare coated cells of endothelial cells, coated cells of fibroblasts, and coated cells of a cell population collected from a cancer patient. Then, a multilayer composed of the coated cells of fibroblasts can be formed, which is then laminated with a single layer formed from the coated cells of endothelial cells, a multilayer formed from the coated cells of fibroblasts, and a single layer formed from the coated cells of the cells containing cancer cells in sequence. This enables production of a cell structure having a vascular network structure inserted between thick fibroblast layers.

Moreover, it is also possible to, for example, separately prepare coated cells of endothelial cells, coated cells of fibroblasts, and coated cells of a cell population collected from a cancer patient. Then, a multilayer composed of the coated cells of fibroblasts can be formed, which is then laminated with a single layer formed from the coated cells of endothelial cells, a multilayer formed from the coated cells of fibroblasts, and a single layer formed from the coated cells of the cells containing cancer cells in sequence. This enables production of a cell structure having a vascular network structure inserted between thick fibroblast layers and having a layer containing cancer cells collected from a cancer patient.

Further, a cell structure can be produced by, for example, using a method including the following steps (A) to (C). In this method, steps (A) and (B) are performed at least once, followed by step (C).
Step (A) of obtaining a mixture in which cells are suspended in a solution containing at least a cationic substance, an extracellular matrix component, and a polyelectrolyte;
step (B) of collecting the cells from the obtained mixture and forming a cell aggregate on a substrate; and
step (C) of culturing the cell aggregate to obtain a cell structure.

In the present specification, the term "cell aggregate" refers to a population of cells, that is, a structure composed of cells combined into a single unit. The cell aggregates also include cell precipitates obtained by centrifugation, filtration, or the like (that is, aggregates of cells formed by precipitating cells). In an embodiment, the cell aggregate is in the form of a slurry-like viscous body. The term "slurry-like viscous body" as used herein refers to a gel-like cell aggregate as described in Akihiro Nishiguchi et al., Cell-cell crosslinking by bio-molecular recognition of heparin-based layer-by-layer nanofilms, Macromol Biosci., 15 (3), 312-317, 2015.

Step (A) is performed by suspending cells that are used to produce the cell structure in a solution containing at least a cationic substance, an extracellular matrix component, and a polyelectrolyte (hereinafter also referred to as a "cell suspension solution"). The cell population suspended in the cell suspension solution may be all or part of the cells that are used to produce the cell structure. When the cell population suspended in the cell suspension solution is all of the cells used to produce the cell structure, it is preferred to obtain a mixture by suspending a cell population containing vasculature endothelial cells and cells other than vasculature endothelial cells and having an N_{E} ratio of 10 to 25% in the cell suspension solution. The N_{E} ratio is the ratio of the number of vasculature endothelial cells to the number of cells other than vasculature endothelial cells. The cells used in the method according to the present embodiment may be the same as the cells constituting the cell structure according to the present invention.

As the cationic substance used in the present embodiment, any substance having a positive charge can be used as long as it does not adversely affect the growth of cells and formation of cell aggregates. Examples of cationic substance include cationic buffer solutions, such as tris-hydrochloric acid buffer solution, tris-maleic acid buffer solution, bis-tris buffer solution and HEPES; ethanolamine, diethanolamine, triethanolamine, polyvinylamine, polyallylamine, polylysine, polyhistidine, polyarginine, and the like. The cationic substance used in the present embodiment is preferably a cationic buffer solution.

The concentration of the cationic substance is not specifically limited as long as it does not adversely affect the growth of cells and formation of cell aggregates. The concentration of the cationic substance used in the present embodiment is preferably 10 mM to 100 mM. The concentration of the cationic substance used in the present embodiment may be, for example, more preferably 20 mM to 90 mM, even more preferably 30 mM to 80 mM, still even more preferably 40 mM to 70 mM, and still even more preferably 45 mM to 60 mM. Further, the concentration of the cationic substance used in the present embodiment is still even more preferably 50 mM.

When a cationic buffer solution is used as the cationic substance, the pH of the cationic buffer solution is not specifically limited as long as it does not adversely affect the growth of cells and formation of cell aggregates. The pH of the cationic buffer solution used in the present embodiment is preferably 6.0 to 8.0. For example, the pH of the cationic buffer solution used in the present embodiment may be 7.0, 7.1, 7.2, 7.3, 7.4, 7.5, 7.6, 7.7, 7.8, 7.9 or 8.0. The pH of the cationic buffer solution used in the present embodiment is more preferably 7.2 to 7.6. The pH of the cationic buffer solution used in the present embodiment is still more preferably 7.4.

In the present invention and the present specification, the term "polyelectrolyte" refers to a polymer with a dissociable functional group in the polymer chain. As the polyelectrolyte used in the present embodiment, any polyelectrolyte can be used as long as it does not adversely affect the growth of cells and formation of cell structure. Examples of the polyelectrolyte include, but are not limited to, glycosaminoglycans such as heparin, chondroitin sulfate (e.g., chondroitin 4-sulfate and chondroitin 6-sulfate), heparan sulfate, dermatan sulfate, keratan sulfate and hyaluronic acid; dextran sulfate, rhamnan sulfate, fucoidan, carrageenan, polystyrene sulfonic acid, polyacrylamido-2-methylpropanesulfonic acid, polyacrylic acid, and the like. The polyelectrolyte may be derivatives of those described above. The mixture prepared in step (A) may be mixed with only one polyelectrolyte, or two or more polyelectrolytes in combination. In production of the cell structure according to the present invention, it is preferred to use glycosaminoglycans, and it is more preferred to use heparin and/or dextran sulfate.

The amount of the polyelectrolyte mixed in the cell suspension solution is not specifically limited as long as it does not adversely affect the growth of cells and production of cell structure. For example, the concentration of the polyelectrolyte in the cell suspension solution may be greater than 0 mg/mL, preferably 0.010 mg/mL or greater, more preferably 0.020 mg/mL or greater, even more preferably 0.025 mg/mL or greater, and still even more preferably 0.05 mg/mL or greater. Further, the concentration of the polyelectrolyte in the cell suspension solution is preferably less than 1.0 mg/mL, more preferably 0.75 mg/mL or less, even more preferably 0.5 mg/mL or less, still even more preferably 0.25 mg/mL or less, and particularly preferably 0.1 mg/mL or less.

As the extracellular matrix component used in the present embodiment, any component that constitutes an extracellular matrix (also referred to as ECM) can be used as long as it does not adversely affect the growth of cells and formation of cell structure. Examples include collagen, laminin, fibronectin, vitronectin, elastin, tenascin, entactin, fibrillin, proteoglycan, glycosaminoglycan, and modifications or variants thereof. Examples of the proteoglycan include chondroitin sulfate proteoglycan, heparan sulfate proteoglycan, keratan sulfate proteoglycan, dermatan sulfate proteoglycan, and the like. Examples of the glycosaminoglycan include hyaluronic acid, chondroitin 4-sulfate, chondroitin 6-sulfate, dermatan sulfate, keratan sulfate, heparan sulfate, heparin, and the like. The extracellular matrix component may also be modified forms or variants of the above components. The mixture prepared in step (A) may be mixed with only one extracellular matrix component, or two or more extracellular matrix components in combination. In production of the cell structure according to the present invention, it is preferred to use one or more selected from the group consisting of collagen, laminin and fibronectin; and it is particularly preferred to use collagen.

The amount of extracellular matrix component mixed in the cell suspension solution is not specifically limited as long as it does not adversely affect the growth of cells and production of cell structure. For example, the concentration of the extracellular matrix component in the cell suspension solution may be greater than 0 mg/mL, preferably 0.010 mg/mL or greater, more preferably 0.020 mg/mL or greater, even more preferably 0.025 mg/mL or greater, and still even more preferably 0.05 mg/mL or greater. Further, the concentration of the extracellular matrix component in the cell suspension solution is preferably less than 1.0 mg/mL, more preferably 0.75 mg/mL or less, even more preferably 0.5 mg/mL or less, still even more preferably 0.25 mg/mL or less, and particularly preferably 0.1 mg/mL or less.

The mixing ratio of the polyelectrolyte to the extracellular matrix component in the cell suspension solution is in the range of 1:2 to 2:1. In production of the cell structure according to the present invention, the mixing ratio of the polyelectrolyte to the extracellular matrix component is preferably in the range of 1:1.5 to 1.5:1, and more preferably 1:1.

The cell structure according to the present invention is produced by performing steps (A) and (B) at least once, and then performing step (C). By repeating steps (A) to (C), a cell structure with sufficient thickness can be produced. Specifically, the following process is repeated: the mixture prepared in step (A) is seeded, as step (B), on the cell structure obtained in previous step (C), and then step (C) is performed. The cell composition of the mixture newly seeded on the cell structure obtained in step (C) may be the same or different from the cell composition that constitute the already produced cell structure.

The method according to the present embodiment may include, after step (A), step (A'-1) of removing the liquid part from the obtained mixture to obtain a cell aggregate, step (A'-2) of suspending the cell aggregate in a solution, and step (B'), as step (B), of precipitating cells from the obtained suspension to form a cell precipitate on the substrate.

In the method according to the present embodiment, the cells and the cell suspension solution may be mixed in a suitable container, such as a dish, tube, flask, bottle or plate, or on the substrate used in step (B). Suspension in step (A'-2) may also be carried out in a suitable container, such as a dish, tube, flask, bottle or plate, or on the substrate used in step (B').

In the method according to the present embodiment, the liquid part can be removed in step (A'-1) using a method known to those skilled in the art. For example, the liquid part may be removed by centrifugation or filtration. The conditions of centrifugation are not specifically limited as long as they do not adversely affect the growth of cells and formation of cell aggregate. For example, a microtube containing the mixture may be subjected to centrifugation at room temperature and 400×g for 1 minute to separate the liquid part from the cell aggregate, thereby removing the liquid part. Alternatively, the liquid part may be removed after collecting the cells by spontaneous sedimentation.

The solution used in step (A'-2) of the above method according to the present embodiment is not specifically limited as long as it does not adversely affect the growth of cells and formation of cell aggregates. For example, a cell culture medium or buffer solution suitable for the cells to be used is used.

Examples of the substrate used in step (B) or (B') of the above method according to the present embodiment include a culture vessel for use in culturing cells. The culture vessel may be a vessel having a material and a shape typically used for cell or microorganism culture. Examples of the material of the culture vessel include, but are not limited to, glass, stainless steel, plastic, and the like. Examples of the culture vessel include, but are not limited to, dishes, tubes, flasks, bottles, plates, and the like. The substrate may be, for example, a material provided in a culture vessel that allows liquid to pass through but does not allow cells in the liquid to pass through.

The substrate used in the present embodiment is preferably a permeable membrane. Examples of culture vessels having a permeable membrane include, but are not limited to, cell culture inserts, such as Transwell (registered trademark) insert, Netwell (registered trademark) insert, Falcon (registered trademark) cell culture insert, and Millicell (registered trademark) cell culture insert.

In the above method according to the present embodiment, the cells can be collected in step (B) or (B') using a method known to those skilled in the art. For example, the cells may be collected by centrifugation, magnetic separation or filtration. The conditions of centrifugation are not specifically limited as long as they do not adversely affect the growth of cells. For example, the cells may be collected by seeding the mixture or suspension in a cell culture insert and centrifuging it at room temperature (specifically 25°C) and 400×g for 1 minute. Alternatively, the cells may be collected by spontaneous sedimentation. In step (B'), for example, the liquid part may be removed from the suspension by centrifugation or filtration to thereby form a cell precipitate on the substrate.

Alternatively, the cell precipitate may be formed on the substrate by spontaneous precipitation. The cell aggregate in step (B) or the cell precipitate in step (B') may be in the form of laminae.

By culturing in step (C) of the method according to the present embodiment, one or more cell structures are produced on the substrate, the surface of which is an endothelial cell layer formed of the vasculature endothelial cells, and the inside of which contains cells other than the vasculature endothelial cells. In step (C), the cells can be cultured under culture conditions suitable for the cells to be cultured. Those skilled in the art can select a suitable medium depending on the cell type and desired function. Various conditions, such as culture temperature and culture time, can also be easily determined by those skilled in the art.

The culture medium for use in the culture in step (C) is not specifically limited as long as it enables growth of cells constituting the cell structure, and may preferably be a medium with low or no growth factor content, such as epidermal growth factor (also called EGF), vascular epidermal growth factor (also called VEGF), fibroblast growth factor (also called FGF), insulin-like growth factor (also called IGF), or the like, or a medium with low or no serum content. If the content of various growth factors is high, angiogenesis may be promoted. In the present embodiment, a medium containing no growth factor is preferred, and a serum-free medium is more preferred.

Further, a cell structure can be produced by, for example, using a method including the following steps (1) to (3).
(1) Step of obtaining a mixture containing cells, a cationic substance, an extracellular matrix component and a polyelectrolyte;
(2) step of gelling the mixture to obtain a gel composition; and
(3) step of incubating the gel composition to obtain a cell structure.

The cells described herein are cells used to form a cell structure, and contain stromal cells in which a gene encoding a live cell marker protein has been knocked out and cancer cells.

Further, the form of the cell structure is not specifically limited, and may be, for example, a cell structure formed by culturing cells in a vessel such as a cell culture insert, a cell structure formed by culturing cells in a scaffold of a natural biopolymer such as collagen or a synthetic polymer, a cell aggregate (also called a spheroid), or a sheet-like cell structure.

Each step will now be described.

First, in step (1), a mixture is obtained by mixing cells, a cationic substance, an extracellular matrix component and a polyelectrolyte. The cells, the cationic substance, the extracellular matrix component and the polyelectrolyte may be mixed in an aqueous solvent. Examples of the aqueous solvent include, but are not limited to, water, buffer solutions and media.

In step (1), stromal cells in which a gene encoding a live cell marker protein has been knocked out and cancer cells are used. The stromal cells in which a gene encoding a live cell marker protein has been knocked out and the cancer cells that are used may be any cells described above. Producing a cell structure using both the stromal cells in which a gene encoding a live cell marker protein has been knocked out and the cancer cells enables production of various cancer model tissues. For example, using NHDF, HUVEC and cancer cells enables production of a cell structure that contains cancer cells and capillaries inside. Using NHDF and colorectal cancer cells enables production of a colorectal cancer model tissue. Further, using NHDF, human iPS cell-derived myocardial cells (also called iPS-CM) and cancer cells enables production of a myocardial cancer model tissue that exhibits synchronous beating.

The ratio of the stromal cells in which a gene encoding a live cell marker protein has been knocked out : cancer cells (number of cells) in step (1) may be 99:1 to 9:1, preferably 80:20 to 50:50, more preferably 20:80 to 50:50, and even more preferably 10:90 to 50:50.

The cationic substance used may be any of those exemplified in the description of step (A). Among them, a cationic buffer solution is preferred, and a tris-HCl buffer solution is more preferred as the cationic substance.

The concentration of the cationic substance in the mixture in step (1) may be any of those exemplified in the description of step (A). Further, the concentration of the cationic substance may be 40 mM to 70 mM, and preferably 45 mM to 60 mM.

When a cationic buffer solution is used as the cationic substance, the pH of the cationic substance may be any of those exemplified in the description of step (A).

The extracellular matrix component may be any of those exemplified in the description of step (A).

The total content of the extracellular matrix component in the mixture in step (1) is not specifically limited as long as it does not adversely affect the growth of cells and formation of gel composition or cell aggregate described later, and may be 0.005 mg/mL or greater and 1.5 mg/mL or less, preferably 0.005 mg/mL or greater and 1.0 mg/mL or less, more preferably 0.01 mg/mL or greater and 1.0 mg/mL or less, even more preferably 0.025 mg/mL or greater and 1.0 mg/mL or less, and still even more preferably 0.025 mg/mL or greater and 0.1 mg/mL or less. The extracellular matrix component may be dissolved in an appropriate solvent before use. Examples of the solvent include, but are not limited to, water, buffer solutions and acetic acid. In particular, buffer solutions or acetic acid are preferred.

In the present specification, the polyelectrolyte used may be any of those exemplified in the description of step (A). The polyelectrolyte is preferably a glycosaminoglycan. In particular, heparin, chondroitin sulfate and dermatan sulfate are preferred, and heparin is particularly preferred.

The concentration of the polyelectrolyte in the mixture in step (1) is not specifically limited as long as it does not adversely affect the growth of cells and formation of gel composition or cell aggregate. Unlike the extracellular matrix component, the polyelectrolyte is effective at any concentration as long as it is below the solubility limit, and does not inhibit the effects of the extracellular matrix component. The concentration of polyelectrolyte is preferably 0.005 mg/mL or greater, more preferably 0.005 mg/mL or greater and 1.0 mg/mL or less, even more preferably 0.01 mg/mL or greater and 1.0 mg/mL or less, still even more preferably 0.025 mg/mL or greater and 1.0 mg/mL or less, and still even more preferably 0.025 mg/mL or greater and 0.1 mg/mL or less.

The polyelectrolyte may be dissolved in an appropriate solvent before use. Examples of the solvent include, but are not limited to, water and buffer solutions. When a cationic buffer solution is used as the above cationic substance, the polyelectrolyte may be dissolved in a cationic buffer solution before use.

A mixing ratio (final concentration ratio) between the polyelectrolyte and the extracellular matrix component in the mixture in step (1) is preferably 1:2 to 2:1, more preferably 1:1.5 to 1.5:1, and even more preferably 1:1.

In step (1), the cells, the cationic substance, the extracellular matrix component and the polyelectrolyte can be mixed in a suitable vessel such as a dish, tube, flask, bottle, well plate or cell culture insert. The mixing may be performed in a vessel used in step (2).

Further, the mixture in step (1) may also contain other components than the cells, the cationic substance, the extracellular matrix component and the polyelectrolyte. Examples of the other components include gelling agents, cell culture media, and the like necessary for obtaining a gel composition in step (2).

Examples of the gelling agent include extracellular matrix components; agarose; pectin; combinations of fibrinogen and thrombin. The gelling agent may be contained in advance in the mixture in step (1), or may be added, in step (2) described below, to the mixture obtained in step (1).

In step (2) subsequent to step (1), the mixture obtained in step (1) is gelled to obtain a gel composition. The vessel used in step (2) may be the same as the vessel used in step (B). The method of gelling depends on the gelling agent used, and, for example, the mixture obtained in step (1) may be subjected to gelling conditions. Alternatively, a gelling agent may be added to the mixture obtained in step (1), and then the mixture may be subjected to gelling conditions.

For example, when an extracellular matrix component is gelled to obtain a gel composition, the gelling conditions include, for example, allowing the mixture obtained in step (1) to stand at about 37°C. As a result, the extracellular matrix component contained in the mixture obtained in step (1) is gelled, whereby a gel composition is obtained. Alternatively, an extracellular matrix component may be added to the mixture obtained in step (1), and then the mixture may be gelled by allowing it to stand at about 37°C.

Further, when agarose is used as the gelling agent, agarose may be added to the mixture obtained in step (1), and, after dissolving the agarose under temperature conditions higher than or equal to the melting point of the agarose used, the mixture may be gelled by allowing it to stand under temperature conditions lower than or equal to the freezing point of the agarose used.

Further, when pectin is used as the gelling agent, pectin may be added to the mixture obtained in step (1). As a result, the pectin is gelled by divalent ions such as calcium ions contained in the mixture, whereby a gel composition is obtained.

Further, fibrinogen and thrombin may be used as the gelling agent. Thrombin, a type of serine protease, cleaves the fibrinogen to form fibrin monomers. The fibrin monomers polymerize with each other by the action of calcium ions to form poorly-soluble fibrin polymers. The fibrin polymers are crosslinked *in vivo* by the action of factor XIII (fibrin stabilizing factor) to form mesh-like fibers called stabilized fibrin, causing blood coagulation. In the present specification, a gel composition which is gelled due to fibrin polymers may also be referred to as a fibrin gel.

That is, step (2) of obtaining a gel composition may include a step of mixing thrombin and fibrinogen with the mixture obtained in step (1). The gel composition in step (2) may contain a fibrin gel as a gel component.

In this case, step (2) of obtaining a gel composition preferably include step (2-1) of adding thrombin to the mixture obtained in step (1), and step (2-2) of adding fibrinogen to the mixture to which thrombin has been added, whereby the fibrin gel is formed to gel the mixture.

When fibrinogen and thrombin are used as the gelling agent, the fibrinogen concentration in the mixture in step (2) is preferably 0.5 mg/mL or greater and 25 mg/mL or less. The concentration of 0.5 mg/mL or greater facilitates gelling when the fibrinogen is mixed with thrombin. The concentration of 25 mg/mL or less facilitates dissolving in the mixture. Further, thrombin is preferably dissolved or dispersed in the mixture in step (2).

If the mixture contains a substance with anticoagulant properties (typically, when heparin is selected as the polyelectrolyte), it is conceivable that fibrin may not be polymerized. For this reason, a substance with anticoagulant properties is not usually used together with fibrin. In the present embodiment, however, polymerization of fibrin is not inhibited, although it is not clear whether this is due to the combination of materials or the concentration being low.

Adding fibrinogen first to the mixture obtained in step (1) may cause the mixture to be gelled depending on the conditions. It is presumed that the fibrinogen is cleaved by some protease contained in the mixture obtained in step (1), and forms fibrin monomers. Therefore, in mixing thrombin and fibrinogen with the mixture obtained in step (1), it is preferred to add thrombin first and then add fibrinogen with the mixture. Alternatively, a gel composition can be obtained by adding only fibrinogen as a gelling agent in step (2).

Thus, the gel composition formed in step (2) may contain a gel component in which at least one of the extracellular matrix component, agarose, pectin and fibrin monomers is gelled.

Subsequently, in step (3), the gel composition obtained in step (2) is incubated to obtain a cell structure. The duration for culturing the gel composition to obtain a cell structure may be 5 minutes to 72 hours. Step (3) promotes adhesion between cells contained in the gel composition, whereby the effect of stabilizing the cell structure is achieved.

In step (3), the cells can be cultured under culture conditions suitable for the cells to be cultured. Those skilled in the art can select a suitable medium depending on the cell type and desired function.

Examples of the medium include, but are not limited to, media such as DMEM, EMEM, MEMα, RPMI-1640, McCoy's 5A and Ham's F-12, and media obtained by adding about 1 vol% to 20 vol% of serum to these media. Examples of serum include bovine serum (CS), fetal bovine serum (FBS), fetal horse serum (HBS), and the like. Conditions such as the temperature and atmospheric composition of the culture environment may also be adjusted according to the cells to be cultured.

The vessel used in step (3) may be the same as the vessel used in step (B). In step (3), the vessel used in step (2) may be used as it is, or another vessel may be used.

Upon cell culture, a substance for suppressing deformation of the obtained cell structure (such as tissue contraction, peeling at the tissue edge, or the like) may be added to the medium. Examples of such a substance include, but are not limited to, Y-27632, which is a Rho-associated coiled-coil forming kinase/Rho binding kinase (ROCK) inhibitor.

Step (3) may be performed after step (1) and step (2) are performed two or more times. By repeating step (1) and step (2), a cell structure having a plurality of layers can be produced. That is, a cell structure having an increased thickness can be produced.

Further, step (1) and step (2) may be repeated using a different cell population for each repetition to form a cell structure composed of different types of cells.

The production method of the present embodiment may further include, before step (2) of obtaining a gel composition, step (1') of applying an external force to the mixture obtained in step (1) to obtain a cell aggregate containing the cells, the cationic substance, the extracellular matrix component and the polyelectrolyte; and, in step (2) of obtaining a gel composition, a step of gelling, as the mixture obtained in step (1), the cell aggregate obtained in step (1') to obtain the gel composition.

That is, a method of producing a cell structure of the present embodiment includes: step (1) of obtaining a mixture containing cells, a cationic substance, an extracellular matrix component and a polyelectrolyte; step (1') of applying an external force to the mixture to obtain a cell aggregate containing the cells, the cationic substance, the extracellular matrix component and the polyelectrolyte; step (2') of gelling the cell aggregate to obtain a gel composition; and step (3) of incubating the gel composition to obtain a cell structure.

The cell aggregate may be formed by allowing the mixture obtained in step (1) to stand in an appropriate vessel, or may be formed by placing the mixture obtained in step (1) in an appropriate vessel and subjecting it to, for example, centrifugation, magnetic separation, filtration, or the like to aggregate the cells. That is, applying an external force in step (1') may refer to allowing the mixture to stand to apply gravity, or performing centrifugation, magnetic separation, filtration, or the like. After the cells are aggregated by standing, centrifugation, magnetic separation, filtration, or the like, the liquid part may be removed or may not be removed.

The vessel used in step (1') may be a culture vessel used for cell culture. The culture vessel may be a vessel having a material and a shape typically used for cell or microorganism culture. Examples of the material of the culture vessel include, but are not limited to, glass, stainless steel, plastic, and the like. Examples of the culture vessel include, but are not limited to, a dish, tube, flask, bottle, well plate, cell culture insert, and the like. Preferably, the vessel is, at least in part, made of a material that allows liquid to pass through but does not allow cells in the liquid to pass through. Examples of such a vessel include, but are not limited to, cell culture inserts, such as a Transwell (registered trademark) insert, Netwell (registered trademark) insert, Falcon (registered trademark) cell culture insert, and Millicell (registered trademark) cell culture insert.

The conditions of centrifugation are not specifically limited as long as they do not adversely affect the growth of cells. For example, the cells can be aggregated to obtain a cell aggregate by placing the mixture in a cell culture insert and centrifuging it at 10°C and 400×g for 1 minute.

Step (2') of gelling the cell aggregate to obtain a gel composition can be performed in the same manner as step (2) of gelling the mixture obtained in step (1) to obtain a gel composition described above.

For example, when an extracellular matrix component is used as the gelling agent, the cell aggregate obtained in step (1') may be allowed to stand at about 37°C, or an extracellular matrix component may be added to the cell aggregate obtained in step (1') and then the cell aggregate may be gelled by allowing it to stand at about 37°C.

Further, when agarose is used as the gelling agent, agarose may be added to the cell aggregate obtained in step (1'), and, after dissolving the agarose under temperature conditions higher than or equal to the melting point of the agarose used, the cell aggregate may be gelled by allowing it to stand under temperature conditions lower than or equal to the freezing point of the agarose used.

Further, when pectin is used as the gelling agent, pectin may be added to the cell aggregate obtained in step (1'). As a result, the pectin is gelled by divalent ions such as calcium ions contained in the mixture, whereby a gel composition is obtained.

Further, fibrinogen and thrombin may be used as the gelling agent. That is, step (2') of obtaining a gel composition may include a step of mixing thrombin and fibrinogen with the cell aggregate obtained in step (1'). The gel composition in step (2') may contain a fibrin gel as a gel component.

In this case, step (2') of obtaining a gel composition preferably include step (2-1') of adding thrombin to the cell aggregate obtained in step (1'), and step (2-2') of adding fibrinogen to the cell aggregate to which thrombin has been added, whereby the fibrin gel is formed to gel the mixture.

Subsequently, in step (3), the gel composition obtained in step (2') is incubated to obtain a cell structure. Step (3) is the same as that described above.

### [Anticancer Drug]

The anticancer drugs used in the assessment method may be drugs used for cancer therapy, and include not only drugs that directly act on cancer cells, such as cytotoxic drugs, but also drugs that are not cytotoxic but suppress the growth of cancer cells, etc. Examples of non-cytotoxic anticancer drugs include: drugs that do not directly attack cancer cells, but exhibit the function to suppress the growth of cancer cells, blunt the activity of cancer cells, or kill cancer cells by the cooperative action with *in vivo* immune cells or other drugs; and drugs that suppress the growth of cancer cells by impairing cells or tissues other than cancer cells. The anticancer drugs used in the present embodiment may be known drugs having anticancer activity, or candidate compounds for novel anticancer drugs.

Examples of the cytotoxic anticancer drugs include, but are not limited to, molecular targeted drugs, alkylating agents, antimetabolites represented by 5-FU-based anticancer drugs, plant alkaloids, anticancer antibiotics, platinum derivatives, hormonal agents, topoisomerase inhibitors, microtubule inhibitors, and compounds classified into biological response modifiers.

Examples of the non-cytotoxic anticancer drugs include, but are not limited to, angiogenesis inhibitors, prodrugs of anticancer drugs, drugs that regulate intracellular metabolism enzyme activity associated with the metabolism of anticancer drugs or prodrugs thereof (hereinafter referred to as "intracellular enzyme regulators" in the specification), immunotherapy agents, and the like. Other examples include drugs that are finally involved in anticancer activity by increasing the function of anticancer drugs or improving the *in vivo* immune function.

The angiogenesis inhibitors may be compounds that are expected to have angiogenesis inhibitory activity, and may be known angiogenesis inhibitors or candidate compounds for novel angiogenesis inhibitors. Examples of known angiogenesis inhibitors include Avastin, EYLEA, Suchibaga, CYRAMZA (registered trademark) (also known as ramucirumab, manufactured by Eli Lilly), BMS-275291 (manufactured by Bristol-Myers), Celecoxib (manufactured by Pharmacia/Pfizer), EMD121974 (manufactured by Merck), Endostatin (manufactured by EntreMed), Erbitaux (manufactured by ImClone Systems), Interferon-α (manufactured by Roche), LY317615 (manufactured by Eli Lilly), Neovastat (manufactured by Aeterna Laboratories), PTK787 (manufactured by Abbott), SU6688 (manufactured by Sugen), Thalidomide (manufactured by Celgene), VEGF-Trap (manufactured by Regeneron), Iressa (registered trademark) (also known as gefitinib, manufactured by AstraZeneca), Caplerusa (registered trademark) (also known as vandetanib, manufactured by AstraZeneca), Recentin (registered trademark) (also known as cediranib, manufactured by AstraZeneca), VGX-100 (manufactured by Circadian Technologies), VD1 and cVE199, VGX-300 (manufactured by Circadian Technologies), sVEGFR2, hF4-3C5, Nexavar (registered trademark) (also known as sorafenib, manufactured by Bayer Yakuhin), Vortrient (registered trademark) (also known as pazopanib, manufactured by GlaxoSmithKline), Sutent (registered trademark) (also known as sunitinib, manufactured by Pfizer), Inlyta (registered trademark) (also known as axitinib, manufactured by Pfizer), CEP-11981 (manufactured by Teva Pharmaceutical Industries), AMG-386 (also known as trebananib, manufactured by Takeda Yakuhin), anti-NRP2B (manufactured by Genentech), Ofev (registered trademark) (also known as nintedanib, manufactured by Boehringer Ingelheim), AMG706 (also known as motesanib, manufactured by Takeda Yakuhin), and the like.

Prodrugs of anticancer drugs are drugs that are converted into active substances having anticancer activity by organs such as the liver or intracellular enzymes of cancer cells. Cytokine networks enhance enzyme activity to thereby increase the number of activators, and result in the enhancement of antitumor effects; thus, these prodrugs are exemplified as drugs involved in anticancer activity.

Examples of intracellular enzyme regulators include gimeracil, which does not have a direct antitumor effect when used alone, but is involved in anticancer activity by inhibiting an enzyme (dihydropyrimidine dehydrogenase: DPD) which degrades 5-FU-based anticancer drugs.

Examples of immunotherapy agents include drugs used for biological response modifier therapy, such as krestin, lentinan and OK-432 (hereinafter abbreviated as "BRM preparations"); cytokine-based preparations, such as interleukins and interferons; and the like.

In the assessment method according to the present embodiment, one anticancer drug may be used, or two or more anticancer drugs may be used in combination. Furthermore, one or more anticancer drugs may be used in combination with drugs other than anticancer drugs. For example, when an anticancer drug that exhibits an anticancer effect when used alone is administered in combination with other drugs in actual clinical practice, the assessment method according to the present embodiment may be performed using the anticancer drug in combination with the other drugs.

### <Anticancer Drug Assessment Step>

In the anticancer drug assessment step, the anticancer drug is assessed using an expression level of the live cell marker protein as an index.

In the cell structure, a gene encoding a live cell marker protein of stromal cells is missing. Therefore, since the live cell marker protein expressed in the cell structure is derived from cancer cells, the number of viable cancer cells can be calculated by measuring the expression level of the live cell marker protein. That is, the anticancer drug assessment step can be said to include a step of measuring the expression level of the live cell marker protein.

Specifically, when the expression level of the live cell marker protein is significantly lower than that when cultured in the absence of the anticancer drug, the anticancer drug is assessed to have an anticancer effect on the cancer cells contained in the cell structure. In contrast, when the expression level of the live cell marker protein is substantially equal or significantly higher than that when cultured in the absence of the anticancer drug, the anticancer drug is assessed to have no anticancer effect on the cancer cells contained in the cell structure.

In the anticancer drug assessment step, when the live cell marker protein is a secretory protein, the expression level of the live cell marker protein is measured using the culture supernatant (hereinafter, also referred to as a "first measurement method").

On the other hand, when the live cell marker protein is not a secretory protein, the expression level of the live cell marker protein is measured by adding a substance that fluoresces or produces color when reacted with the live cell marker protein, and measuring the expression level of the live cell marker protein based on the amount of fluorescence or coloration of the substance (hereinafter, also referred to as a "second measurement method").

In the anticancer drug assessment step, as described above, the expression level of the live cell marker protein is measured without destroying the cell structure. Accordingly, data can be obtained continuously and over time while maintaining the culture of the cell structure. Therefore, according to the assessment method of the present embodiment, it is possible to assess components that behave quickly, such as metabolites of cancer cells.

### [First Measurement Method]

When the live cell marker protein is a secretory protein, the expression level of the live cell marker protein contained in the culture supernatant can be measured by known protein detection methods such as Western blotting method using a substance that binds specifically to the live cell marker protein, enzyme-linked immunosorbent assay (ELISA), chemiluminescent enzyme immunoassay (CLEIA) and immunoprecipitation.

In the Western blotting method, for example, a culture supernatant containing a live cell marker protein is first prepared as a sample, and the live cell marker protein is separated by acrylamide gel electrophoresis. Next, the acrylamide gel after the separation is transferred to a membrane and reacted with an antibody (hereinafter, referred to as a primary antibody) that can recognize the live cell marker protein. Next, after the primary antibody that has not bound to the live cell marker protein is removed by washing or the like, an immune complex of the generated live cell marker protein and the primary antibody is detected using a labeled secondary antibody. The amount of the live cell marker protein present can be determined by measuring the amount of the labeled secondary antibody that has bound to the generated immune complex.

Examples of the substance that binds specifically to the live cell marker protein include specific antibodies to the live cell marker proteins (hereinafter, also referred to as "live cell marker protein-specific antibodies") and aptamers.

The live cell marker protein-specific antibody may be a polyclonal antibody or a monoclonal antibody, or may be a functional fragment of antibody. In particular, the live cell marker protein-specific antibody may preferably be a monoclonal antibody due to high specificity and excellent quantitation.

The live cell marker protein-specific antibody may be commercially available or may be produced using the method described below.

When the live cell marker protein-specific antibody is a polyclonal antibody, the antibody can be obtained by immunizing immunization animals with antigen (for example, a live cell marker protein or fragments thereof, or cells expressing them) and purifying the antiserum by conventional means (for example, salt precipitation, centrifugation, dialysis or column chromatography, etc.).

The live cell marker protein used as the antigen or a peptide fragment having part of its amino acid sequence may be chemically synthesized or produced by known genetic engineering techniques based on the known base sequence information of the live cell marker protein.

Further, when the live cell marker protein-specific antibody is a monoclonal antibody, the antibody can be produced by a hybridoma technique or recombinant DNA technique.

The term "bind specifically" as used herein means that the antibody binds only to the target protein (i.e., antigen), and can be quantified, for example, by binding of the antibody to an epitope of the antigen in an *in vitro* assay, preferably a plasmon resonance assay using purified wild-type antigen (e.g., BIAcore, GE-Healthcare Uppsala, Sweden, etc.). The binding affinity can be specified by ka (rate constant for antibody binding from the antibody-antigen composite), kD (dissociation constant) and KD (kD/ka). The binding affinity (KD) when an antibody binds specifically to an antigen is preferably 10⁻⁸ mol/L or less, and more preferably 10⁻¹³ mol/L or greater and 10⁻⁹ mol/L or less.

In the present embodiment, "polyclonal antibody" refers to antibody preparation containing different antibodies to different epitopes. That is, when the antibody is a polyclonal antibody, it can contain different antibodies that bind specifically to the live cell marker protein.

Further, "monoclonal antibody" refers to an antibody (including antibody fragments) obtained from a population of substantially homogeneous antibodies. In contrast to the polyclonal antibody, a monoclonal antibody refers to one that recognizes a single determinant on the antigen.

In the present embodiment, a "functional fragment" of an antibody refers to a portion (also called a partial fragment) of an antibody that specifically recognizes a target protein. Specific examples include Fab, Fab', F(ab')2, variable region fragment (Fv), disulfide-linked Fv, single chain Fv (scFv), sc (Fv)2, diabody, multispecific antibody, and polymers thereof.

An aptamer is a substance having a specific binding ability to a target substance. Examples of the aptamer include nucleic acid aptamers, peptide aptamers, and the like. A nucleic acid aptamer having a capability of binding specifically to an antigen can be selected by, for example, the systematic evolution of ligand by exponential enrichment (SELEX) method, or the like. Peptide aptamers having a capability of binding specifically to an antigen can be selected, for example, by a two-hybrid method using yeast, or the like.

Further, the substance that binds specifically to the live cell marker protein may be bound to a labeling substance or a modifying substance.

Examples of the labeling substance for the antibody include stable isotopes, radioisotopes, fluorescent substances, enzymes and magnetic substances. Among these, fluorescent substances and enzymes are preferred as the labeling substance for the antibody since they are easily detectable and highly sensitive. When the substance that binds specifically to the live cell marker protein includes the labeling substance, the live cell marker protein can be detected and quantified easily with high sensitivity.

Examples of the stable isotopes include, but are not limited to, ¹³C, ¹⁵N, ²H, ¹⁷O and ¹⁸O.

Examples of the radioisotopes include, but are not limited to, ³H, ¹⁴C, ¹³N, ³²P, ³³P and ³⁵S.

Examples of the fluorescent substances include, but are not limited to, cyanine dyes (e.g., Cy3 and Cy5), rhodamine 6G reagents and other known fluorescent dyes (e.g., GFP, FITC (Fluorescein) and TAMRA).

Examples of the enzymes include alkaline phosphatase and peroxidase (e.g., HRP). When the labeling substance is an enzyme, it is preferred to use an enzyme substrate. When the enzyme is alkaline phosphatase, p-nitropheny phosphase (pNPP), 4-methylumbelliferyl phosphate (4-MUP), or the like can be used as the enzyme substrate, and when the enzyme is peroxidase, 3,3'-diaminobenzidine (DAB), 3,3',5,5'-tetramethylbenzidine (TMB), o-phenylenediamine (OPD), 2,2-azino-di-(3-ethylbenzothiazoline-6-sulfonic acid) (ABTS) and 10-acetyl-3,7-dihydroxyphenoxazine (ADHP), or the like can be used.

Examples of the magnetic substances include, but are not limited to, gadolinium, Gd-DTPA, Gd-DTPA-BMA, Gd-HP-DO3A, iodine, iron, iron oxide, chromium, manganese, and complexes or chelate complexes thereof.

Examples of the modifying substance include biotin and Fc fragments. When the substance that binds specifically to the live cell marker protein includes the modifying substance, the live cell marker protein can be detected and quantified easily with high sensitivity using a specific binding substance for the modifying substance to which a labeling substance is bound (e.g., a specific antibody, or avidin or streptavidin for a specific binding substance for biotin).

Further, the expression level of the live cell marker protein in the culture supernatant can also be measured by a method of detecting mRNA encoding the live cell marker protein or a nucleotide fragment having a partial sequence thereof. More specifically, examples of the method include, but are not limited to, quantitative RT-PCR, northern blotting and in situ hybridization.

These detection methods can use probes, primers or combinations thereof prepared based on known base sequence information of the gene encoding the live cell marker protein.

### [Second Measurement Method]

When the live cell marker protein is an enzyme rather than a secretory protein, a substrate can be used as the substance that fluoresces or produces color when reacted with the live cell marker protein. A chromogen is bound to the substrate, and upon reaction with the enzyme, which is the live cell marker protein, the chromogen dissociates to cause luminescence (i.e., fluorescence) or produce color (i.e., coloration). The chromogen is not specifically limited as long as it causes luminescence (i.e., fluorescence) or produces color when dissociating from the substrate.

Further, when the live cell marker protein is expressed intracellularly, the substrate preferably has cell membrane permeable properties.

Specific examples of such substrate include glycyl-phenylalanyl-aminofluorocoumarin (also called GF-AFC) when the live cell marker protein is TPP1. Commercially available assay kits containing the GF-AFC include, for example, CellTiter-Fluor^{™} Cell Viability Assay manufactured by Promega.

According to the assessment method of the present embodiment, a cell structure is used which contains a stroma similar to the peripheral tissues of cancer cells in the actual living body, in which components secreted from the stroma have an influence on the cancer cells. Furthermore, it is also possible to use a cell structure containing, in addition to a stroma, a vascular network structure similar to the vasculature structure in the actual living body. Thus, according to the assessment method of the present embodiment, assessment is performed in an environment reproduced *in vitro* closer to the *in vivo* environment; therefore, highly reliable assessment of drug efficacy can be obtained. Anticancer drugs that are assessed to have anticancer effects by the assessment method of the present embodiment can be expected to exhibit sufficient anticancer effects when they are actually administered to cancer patients. Similarly, the combination of an angiogenesis inhibitor and a cytotoxic anticancer drug assessed to have a combination effect by the assessment method of the present embodiment is expected to exhibit an anticancer effect higher than the anticancer drug alone when they are actually administered to cancer patients. Accordingly, the assessment method of the present embodiment can be used as an unprecedented *in vitro* drug assessment tool, for example, in screening or drug-repositioning screening of anticancer drug candidate compounds in the drug design field, or in screening and determination (anticancer drug sensitivity test) of anticancer drug therapy (single drug or combined use of drugs) in the clinical field. In particular, anticancer drugs that are assessed to have anticancer effects by the assessment method of the present embodiment performed using a cell structure containing cancer cells collected from a cancer patient are expected to exhibit suitable anticancer effects when they are actually administered to the cancer patient.

### <Modifications>

In the assessment method of the present embodiment, the effect of combined use of a cytotoxic anticancer drug and an angiogenesis inhibitor can also be assessed using, as the cell structure, a cell structure containing multilayer cells in which a vascular network structure is formed, and some of the component cells are cancer cells, and using the cytotoxic anticancer drug and the angiogenesis inhibitor as anticancer drugs. Since the method uses a cell structure containing a vascular network structure in a three-dimensional structure closer to the *in vivo* state, drug efficacy obtained by the combined use of an angiogenesis inhibitor and an anticancer drug can be accurately assessed without using animal models.

When assessing the effect of combined use of a cytotoxic anticancer drug and an angiogenesis inhibitor, the expression level of the live cell marker protein after the culture step is used as an indicator to assess the anticancer effect of combined use of the angiogenesis inhibitor and the anticancer drug. Specifically, when the expression level of the live cell marker protein is significantly lower than that when cultured in the presence of the anticancer drug alone, it is assessed that the anticancer drug, when used in combination with the angiogenesis inhibitor, provides a higher anticancer effect than when the anticancer drug is used alone, that is, it is assessed that a combination effect is obtained. On the other hand, when the expression level of the live cell marker protein is substantially the same as that when cultured in the presence of the anticancer drug alone, it is assessed that the anticancer drug, when used in combination with the angiogenesis inhibitor, does not provide a higher anticancer effect than when the anticancer drug is used alone, that is, it is assessed that a combination effect is not obtained. Furthermore, when the expression level of the live cell marker protein is significantly larger than that when cultured in the presence of the anticancer drug alone, it is assessed that the anticancer effect is rather reduced by using the anticancer drug in combination with the angiogenesis inhibitor.

The cytotoxic anticancer drugs may be compounds that are expected to have cytotoxicity, and may be known anticancer drugs or candidate compounds for novel anticancer drugs.

Specifically, the assessment method according to this modification includes a culture step of culturing a cell structure containing cancer cells and having a vascular network structure in the presence of an angiogenesis inhibitor and a cytotoxic anticancer drug, and an assessment step of assessing the anticancer effect of combined use of the angiogenesis inhibitor and the anticancer drug using, as an indicator, the expression level of the live cell marker protein in the cell structure after the culture step.

In another aspect, the method of measuring the expression level of the live cell marker protein in this modification includes a culture step of culturing a cell structure containing cancer cells and having a vascular network structure in the presence of an angiogenesis inhibitor and a cytotoxic anticancer drug, and a measurement step of measuring the expression level of the live cell marker protein in the cell structure after the culture step.

The cell structure has a vascular network structure, and is composed of endothelial cells that constitute vessels, and cells that do not constitute vessels (i.e., cells other than endothelial cells).

The type of cells other than endothelial cells contained in the cell structure is not specifically limited as long as they do not inhibit the endothelial cells form forming a vessel network. The cell type can be suitably selected in consideration of the origin and type of cancer cells contained, the types of angiogenesis inhibitor and anticancer drug used for assessment, the *in vivo* environment where the target anticancer activity is exhibited, and the like.

### <<Anticancer Drug Assessment Kit>>

An anticancer drug assessment kit of the present embodiment (hereinafter, also simply referred to as a "kit of the present embodiment") includes a cell structure containing stromal cells in which a gene encoding a live cell marker protein has been knocked out, and a cell culture vessel that accommodates the cell structure.

The assessment method described above can be performed more simply by using an anticancer drug assessment kit, which is a kit including reagents and the like used for the assessment method described above.

For example, the cell structure contained in the kit of the present embodiment may be a cell structure that does not contain cancer cells and has a vascular network structure.

The cell structure contained in the kit of the present embodiment may have a thickness of preferably 5 µm or greater.

The cell structure contained in the kit of the present embodiment may be a cell structure containing cancer cells; however, the kit may contain a cell structure that does not contain cancer cells but contains cells that constitute a stroma, and a cancer cell layer may be formed on the surface of the cell structure immediately before the assessment method is actually performed. Furthermore, the kit may contain, in place of the cell structure, cells other than cancer cells among the cells constituting the cell structure.

The cell structure contained in the kit of the present embodiment contains at least stromal cells, and may contain a semipermeable membrane on a top surface thereof. The cell structure contained in the kit may be one in which a cancer cell layer is laminated on a semipermeable membrane; however, the kit may contain a cell structure that does not contain cancer cells, and that has a semipermeable membrane placed on a cell layer containing stromal cells, and a cancer cell layer may be formed on the semipermeable membrane of the cell structure immediately before the assessment method is actually performed.

The kit may further contain other substances used in the assessment method. Examples of the other substances include anticancer drugs, culture media for the cell structure, reagents for detecting the live cell marker protein (e.g., substances that fluoresces or produces color when reacted with the live cell marker protein), substances used in production of the cell structure (e.g., cationic buffer solutions, strong electrolyte polymers and extracellular matrix components), and the like. The other substances described herein are the same as those described in the above embodiment, and the description thereof will be omitted. The kit may further include a written procedure describing the procedure for performing the above-mentioned anticancer drug assessment method.

Furthermore, when a cytotoxic anticancer drug and an angiogenesis inhibitor are used in combination as anticancer drugs, the effect of combined use of the cytotoxic anticancer drug and the angiogenesis inhibitor can be assessed more simply.

### [Examples]

The present invention will be described below using the examples, but the present invention should not be limited to the examples below.

### [Example 1]

A cell structure containing fibroblasts in which the TPP1 gene had been knocked out and cancer cells was used to assess the anticancer effects of the anticancer drugs, 5-fluorouracil (5-FU) and trametinib. The cell culture vessel used was a Transwell cell culture insert (product number: #3391, manufactured by Corning), and the culture medium used was D-MEM (043-30085, manufactured by FUJIFILM Wako Pure Chemical Corporation) containing 10 volume% fetal bovine serum (FBS) (PO241817, manufactured by Thermo Fisher Scientific).

### 1. Preparation of Fibroblasts in Which TPP1 Gene Has Been Knocked Out

Human neonatal dermal fibroblasts (CC-2509, Normal Human Dermal Fibroblasts: NHDF, manufactured by Lonza) were treated using a TPP1 Human Gene Knockout Kit (KN404471, manufactured by Origene) according to a predetermined protocol to obtain NHDF in which the TPP1 gene had been knocked out (hereinafter, also referred to as "TPP1KONHDF").

### 2. Preparation of Cell Structure

First, 2×10⁶ cells of TPP1KONHDF were suspended in a tris-HCl buffer solution (166-23555, manufactured by FUJIFILM Wako Pure Chemical Corporation) (0.1 mg/mL heparin, 0.1 mg/mL collagen, 50 mM tris, pH7.4) containing heparin (H3149-100KU, manufactured by Sigma) and collagen (ASC-1-100-100, manufactured by Sigma) to prepare a cell suspension. The cell suspension was centrifuged at room temperature (about 25°C) and 400×g for 1 minute to remove the supernatant, and then resuspended in an appropriate amount of culture medium. Then, after the cell suspension was seeded in a Transwell cell culture insert, the Transwell cell culture insert was centrifuged at room temperature (about 25°C) and 400×g for 1 minute. Subsequently, an appropriate amount of culture medium was added to the Transwell cell culture insert, followed by culture in a CO₂ incubator (37°C, 5 v/v% CO₂) for 96 hours. In addition, a sample using normal NHDF was also prepared as a control.

### 3. Seeding of Cancer Cells

1×10³ cells of HT29 (ATCC number: HTB-38TM), a human colorectal adenocarcinoma cell line, were seeded on the top surface of the culture obtained in the above "2." and cultured at 37°C and 5 v/v% CO₂ for 4 days to obtain a cell structure.

### 4. Drug Efficacy Assessment

Next, on day 4 of culture after seeding of the cancer cells, the culture medium was replaced with a culture medium containing 5-FU (S1209, manufactured by Selleck) at a final concentration of 1, 10, 100 or 1000 µM, or trametinib (S2807, manufactured by Selleck) at a final concentration of 0.01, 0.1, 1 or 10 µM, which was then cultured at 37°C and 5 v/v%CO₂ for 72 hours. A sample cultured under conditions without the addition of 5-FU and trametinib was also prepared as a control.

Next, on day 7 of culture after seeding of the cancer cells, the number of viable cells was measured using a CellTiter-Fluor ^{™} Cell Viability Assay (G6080, manufactured by Promega). From the measurement result, the cell viability was calculated using the following formula. The results are shown in Table 1 (5-FU) and Table 2 (trametinib). Hereinafter, the drug efficacy assessment according to the above method may also be referred to as "drug efficacy assessment 1." (Cell viability) (%) = {(number of viable cells under condition of each concentration of anticancer drug) / (number of viable cells under condition of no anticancer drug)} × 100

In addition, the drug efficacy was also assessed using a conventional immunostaining method as a control.

Specifically, on day 4 of culture after seeding of the cancer cells, the culture medium was replaced with a culture medium containing 5-FU (S1209, manufactured by Selleck) at a final concentration of 1, 10, 100 or 1000 µM, or trametinib (S2807, manufactured by Selleck) at a final concentration of 0.01, 0.1, 1 or 10 µM, which was then cultured at 37°C and 5 v/v%CO₂ for 72 hours. A sample cultured under conditions without the addition of 5-FU and trametinib was also prepared as a control.

Next, on day 7 of culture after seeding of the cancer cells, the tissue was fixed and stained by immunofluorescence staining using Alexa 647-conjugated anti-EpCAM antibody, and the stained cells were taken as cancer cells. The area of fluorescence derived from the cancer cells was calculated using a fluorescence mode (Alexa 647 fluorescence detection) of a microscope system (Operetta CLS, manufactured by PerkinElmer). From the measurement result, the cell viability was calculated using the following formula. The results are shown in Table 1 (5-FU) and Table 2 (trametinib). Hereinafter, the drug efficacy assessment according to the above method may also be referred to as "drug efficacy assessment 2 (conventional method)." (Cell viability) (%) = {(area of fluorescence derived from cancer cells under condition of each concentration of anticancer drug) / (area of fluorescence derived from cancer cells under condition of no anticancer drug)} × 100

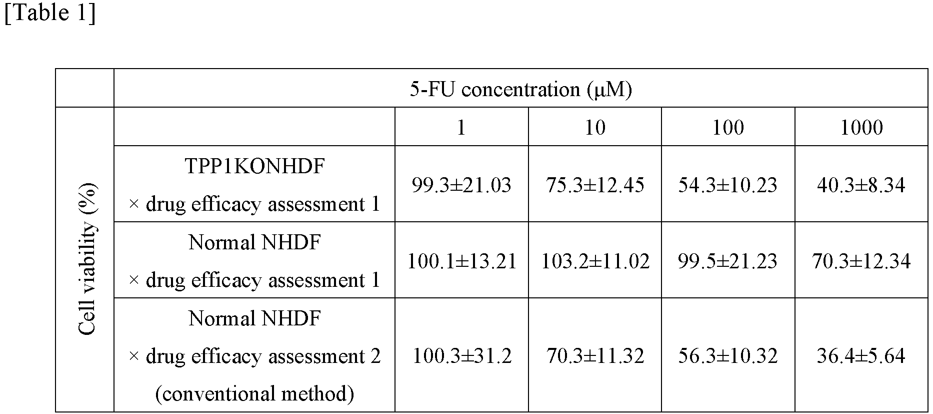

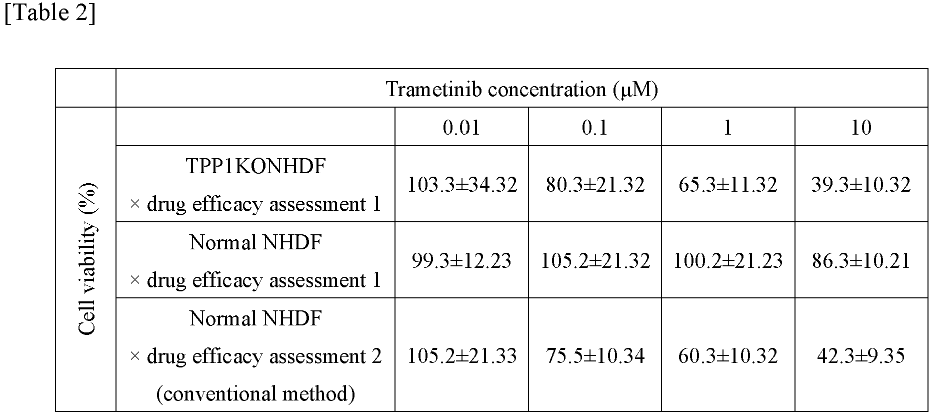

As shown in Tables 1 and 2, in the anticancer drug assessment method of the present embodiment (TPP1KONHDF × drug efficacy assessment 1), both of the two types of anticancer drugs show a drug concentration-dependent decrease in the cell viability, which is substantially the same as the results of the conventional drug efficacy assessment method (normal NHDF × drug efficacy assessment 2 (conventional method)).

Therefore, it is suggested that the anticancer drug assessment method of the present embodiment enables anticancer drug assessment substantially similar to the conventional drug efficacy assessment method in a simple and nondestructive manner.

### [Industrial Applicability]

According to the anticancer drug assessment method of the present embodiment, accurate and nondestructive anticancer drug assessment can be performed using an *in vitro* system. Since it is a nondestructive assessment system, a larger amount of data can be obtained continuously from the same sample over a long period of time.

## Claims

1. An anticancer drug assessment method comprising the steps of:
culturing a cell structure containing stromal cells in which a gene encoding a live cell marker protein has been knocked out and cancer cells in the presence of an anticancer drug; and
assessing the anticancer drug using an expression level of the live cell marker protein as an index in this order, wherein
in the assessment, the expression level of the live cell marker protein is measured using a culture supernatant, or
in the assessment, a substance that fluoresces or produces color when reacted with the live cell marker protein is added, and the expression level of the live cell marker protein is measured based on the amount of fluorescence or coloration of the substance.

2. The anticancer drug assessment method according to claim 1, wherein
the live cell marker protein is a serine protease.

3. The anticancer drug assessment method according to claim 2, wherein
the serine protease is tripeptidyl peptidase 1.

4. The anticancer drug assessment method according to claim 2 or 3, wherein
the substance is a substrate.

5. An anticancer drug assessment kit comprising:
a cell structure containing stromal cells in which a gene encoding a live cell marker protein has been knocked out; and
a cell culture vessel that accommodates the cell structure
